(19) Europäisches Patentamt
European Patent Office
Office européen des brevets

(11) Numéro de publication: **0 428 423 A2**

(12) **DEMANDE DE BREVET EUROPEEN**

(21) Numéro de dépôt: **90402920.4**

(22) Date de dépôt: **17.10.90**

(51) Int. Cl.5: **C07C 43/20**, A61K 31/085, A61K 31/215, A61K 31/255, A61K 31/66, C07C 69/017, C07C 233/65, C07C 43/23, C07C 309/63, C07F 9/09

La demande, qui etait incomplète au moment du dêpot, est publiée telle quelle (article 93 (2) CBE). Le passage de la description ou des revendications qui comporte manifestement une omission est présenté comme tel.

(30) Priorité: **18.10.89 FR 8913607**

(43) Date de publication de la demande: **22.05.91 Bulletin 91/21**

(84) Etats contractants désignés: **AT BE CH DE DK ES FR GB GR IT LI LU NL SE**

(71) Demandeur: **LIPHA, LYONNAISE INDUSTRIELLE PHARMACEUTIQUE**
**115 Avenue Lacassagne**
**F-69003 Lyon(FR)**

(72) Inventeur: **Moinet, Gérard**
**15 Rue Lamartine**
**F-91400 Orsay(FR)**
Inventeur: **Imbert, Thierry**
**8 Hameau Levrieres**
**F-78590 Noisy Le Roi(FR)**
Inventeur: **Marais, Dominique**
**5 Chemin des Pouillères**
**F-78250 Meulan(FR)**
Inventeur: **Vidaluc, Jean Louis**
**28 Boulevard du Général Giraud**
**F-81100 Castres(FR)**
Inventeur: **Mesangeau, Didier**
**5 Rue Auguste Renoir**
**F-77380 Combs La Ville(FR)**

(74) Mandataire: **CABINET GEFIB**
**59 Rue Edouart Vaillant**
**92300 Levallois Perret(FR)**

(54) **Nouveaux aryloxy alcoyl benzènes, leurs procédés de préparation et les compositions pharmaceutiques en renfermant.**

(57) La présente invention a pour objet de nouveaux aryloxy alcoyl benzènes, leurs procédés de préparation ainsi que les compositions pharmaceutiques en renfermant.

Elle a plus particulièrement pour objet de nouveaux phénoxy éthylbenzènes substitués sur l'un ou l'autre noyau et spécifiquement les phénoxy éthylbenzènes substitués de formule générale I

$$Ar - \overset{\displaystyle R}{\overset{|}{CH}} - \overset{\displaystyle R_1}{\overset{|}{CH}} - O - Ar \qquad (I)$$

dans laquelle Ar est un radical aromatique mono- ou bicyclique hydroxylé choisi parmi les dérivés benzéniques de formule

EP 0 428 423 A2

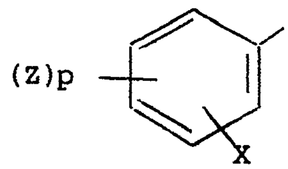

dans laquelle X représente un hydroxyle libre, estérifié par un acide carboxylique, ou un acide sulfonique, par l'acide phosphorique, ou par l'acide sulfurique ou ethérifié par un alcoyle inférieur ou un alcényle inférieur
Z est un substituant choisi dans le groupe constitué par l'hydrogène, les halogènes, un hydroxy, un alcoxy inférieur, un acyloxy, un phényl alcoyle inférieur, un alcoyle inférieur, un alcoyle inférieur substitué par un (alcoyl inférieur) oxycarbonyle ou par un hydroxy carbonyle, une chaine $CO(CH_2)_m CH_3$ dans laquelle m représente un nombre entier allant de 1 à 4, une chaine $CHOH-(CH_2)_{m'} CH_3$ dans laquelle m, représente un nombre entier variant de 1 à 4, un phényle, un phénoxy et un groupe carboxamido, éventuellement substitué par un ou deux radicaux alcoyle inférieur et parmi les dérivés naphtaléniques choisis dans le groupe constitué parmi les composés de formule

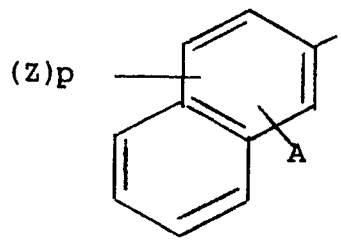

dans laquelle A est un hydroxyle libre, ethérifié ou estérifié
Z est défini comme précédemment
et p est un nombre entier variant de 1 à 3
et ceux de formule

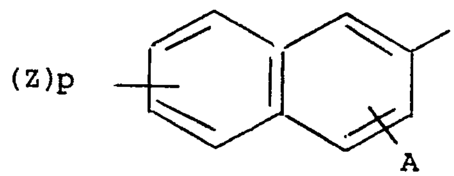

dans laquelle A, Z et p sont définis comme précédemment
Ar′ est un radical aromatique mono- ou bicyclique choisi dans le groupe constitué parmi
- les dérivés benzéniques de formule

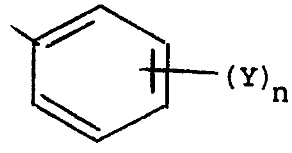

dans laquelle Y représente de l'hydrogène, un halogène, un alcoylène dioxy, un hydroxy, un alcoxy inférieur, un acyloxy dérivé d'un acide organique carboxylique, un alcoyle inférieur, un alcoyle inférieur substitué par un radical (alcoxy inférieur) carbonyle, une chaine oxo alcoyle de formule $CO(CH_2)_m-CH_3$ dans laquelle m représente un nombre entier allant de 1 à 4 et une chaine hydroxy alcoyle de formule $CHOH-(CH_2)_{m'}-CH_3$ dans laquelle m′ représente un nombre entier allant de 1 à 4.
- les dérivés naphtaléniques choisis dans le groupe constitué par les dérivés de formule

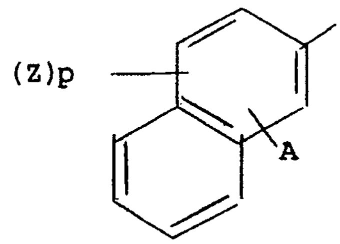

dans laquelle Y est défini comme précédemment
et n est un nombre entier variant de 1 à 3
- et les dérivés de formule

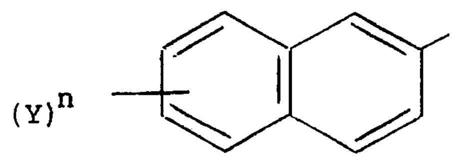

dans laquelle Y est défini comme précédemment
et n est un nombre entier variant de 1 à 3
R est un hydrogène, un hydroxyle, l'oxygène d'une fonction carbonylée ou un radical alcoyle inférieur
$R_1$ est de l'hydrogène ou un radical alcoyle inférieur.

Ces composés servent de principe actif pour des médicaments anti-hypertenseurs, diurétiques et anti-agrégants plaquettaires.

## NOUVEAUX ARYLOXY ALCOYL BENZENES, LEURS PROCEDES DE PREPARATION ET LES COMPOSITIONS PHARMACEUTIQUES EN RENFERMANT

La présente invention a pour objet de nouveaux aryloxy alcoyl benzènes, leurs procédés de préparation ainsi que les compositions pharmaceutiques en renfermant.

Elle a plus particulièrement pour objet nouveaux phenoxy éthylbenzènes substitués sur l'un ou l'autre noyau et spécifiquement les phénoxy éthylbenzènes substitués de formule générale I

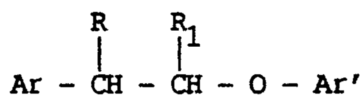

(I)

dans laquelle Ar est un radical aromatique mono- ou bicyclique hydroxylé choisi parmi les dérivés benzéniques de formule

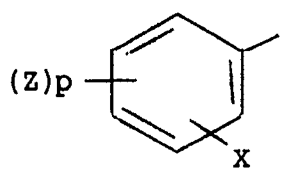

dans laquelle X représente un hydroxyle libre, estérifié par un acide carboxylique, ou un acide sulfonique, par l'acide phosphorique, ou par l'acide sulfurique ou ethérifié par un alcoyle inférieur ou un alcényle inférieur

Z est un substituant choisi dans le groupe constitué par l'hydrogène, les halogènes, un hydroxy, un alcoxy inférieur, un acyloxy, un phényl alcoyle inférieur, un alcoyle inférieur, un alcoyle inférieur substitué par un (alcoyl inférieur) oxycarbonyle ou par un hydroxy carbonyle, une chaine $CO(CH_2)_m CH_3$ dans laquelle m représente un nombre entier allant de 1 à 4, une chaine $CHOH-(CH_2)_{m'} CH_3$ dans laquelle $m'$ représente un nombre entier variant de 1 à 4, un phényle, un phénoxy et un groupe carboxamido, éventuellement substitué par un ou deux radicaux alcoyle inférieur et parmi les dérivés naphtaléniques choisis dans le groupe constitué par les composés de formule

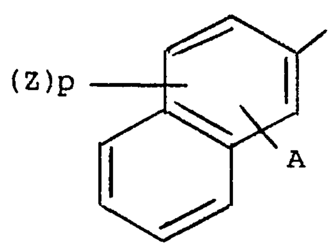

dans laquelle A est un hydroxyle libre, ethérifié ou estérifié

Z est défini comme précédemment

et p est un nombre entier variant de 1 à 3

et ceux de formule

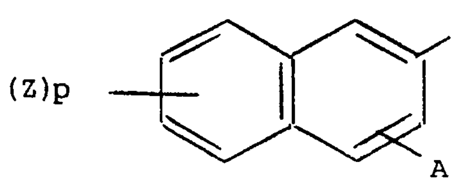

dans laquelle A, Z et p sont définis comme précédemment

Ar′ est un radical aromatique mono- ou bicyclique choisi dans le groupe constitué parmi
- les dérivés benzéniques de formule

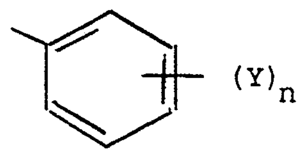

dans laquelle Y représente de l'hydrogène, un halogène, un alcoylène dioxy, un hydroxy, un alcoxy inférieur, un acyloxy dérivé d'un acide organique carboxylique, un alcoyle inférieur, un alcoyle inférieur substitué par un radical (alcoxy inférieur) carbonyle, une chaine oxo alcoyle de formule $CO(CH_2)_m-CH_3$ dans laquelle m représente un nombre entier allant de 1 à 4 et une chaine hydroxy alcoyle de formule $CHOH-(CH_2)_{m'}-CH_3$ dans laquelle m′ représente un nombre entier allant de 1 à 4.
- les dérivés naphtaléniques choisis dans le groupe constitué par les dérivés de formule

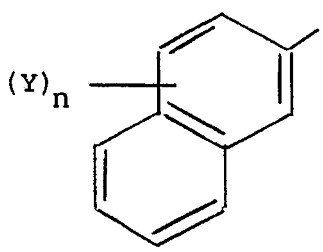

dans laquelle Y est défini comme précédemment
et n est un nombre entier variant de 1 à 3
- et les dérivés de formule

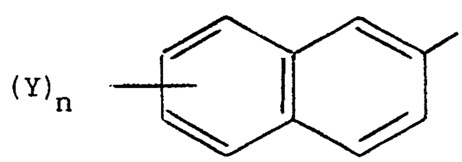

dans laquelle Y est défini comme précédemment
et n est un nombre entier variant de 1 à 3
R est un hydrogène, un hydroxyle, l'oxygène d'une fonction carbonylée ou un radical alcoyle inférieur
$R_1$ est un hydrogène ou un radical alcoyle inférieur

Elle a plus particulièrement pour objet les phénoxyethyl benzènes de formule générale $I_A$

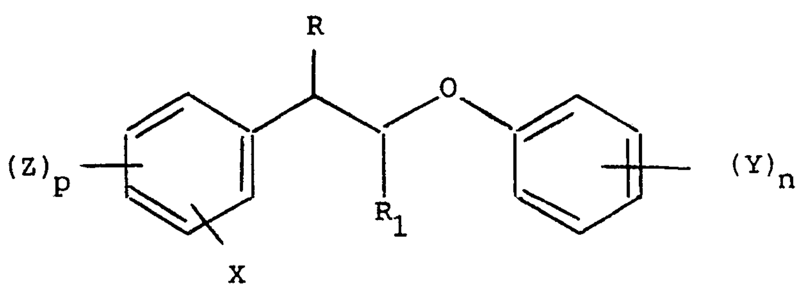

$(I_A)$

dans laquelle X représente un hydroxyle, un alcyloxy dont le reste acyle dérive d'un acide carboxylique, sulfonique, phosphorique, de l'acide sulfurique et de l'acide phosphorique, un alcoxy inférieur ou un alcenyloxy inférieur

Y représnte de l'hydrogène, un halogène, un alcoylène dioxy, un hydroxy, un alcoxy, un acyloxi dérivé d'un acide organique substitué par un alcoxy carbonyle, un oxo alcoyle ayant au moins deux atomes de carbone, un hydroxy alcoyle, un alcoxy carbonyle ou la chaine alcoylidène d'un cycle aromatique ayant 5 ou 6 chainons

Z représente de l'hydrogène, un hydroxyle, un alcoxy, un alcényloxy, un phénoxy, un phénylalcoyle inférieur, un alcoxy carbonyloxy, la chaine alcoylidène d'un homocycle carboné saturé ou aromatique ayant 5 ou 6 chainons, un alcoyle inférieur, un alcoylène dioxy, un acyloxy, un halogène, un alcoxy carbonyle, un amino carbonyle, un carboxyle

n est égal à 1, 2 ou 3

p est égal à 0, 1 ou 2

R est un hydrogène, un hydroxyle, l'oxygène d'une fonction cetone ou un alcoyle inférieur

$R_1$ est d l'hydrogène ou un radical alcoyle inférieur

L'invention comprend aussi :

a) Les formes optiquement-actives des composés de formule générale I lorsque R ou $R_1$ sont différents de l'hydrogène et que R ou $R_1$ représentent un hydroxyle ou un radical alcoyle inférieur.

b) Les sels avec un métal alcalin ou alcalino terreux ou avec une base organique des composés de formule I lorsque X représente un hydroxyle estérifié par l'acide phosphorique ou sulfurique ou les phénates obtenus par neutralisation de la fonction phénol libre par une base alcaline, alcalino-terreuse ou organique.

Parmi les composés selon l'invention, on pourra citer également les composés répondant aux formules suivantes :

- les composés répondant à la formule générale $I_B$

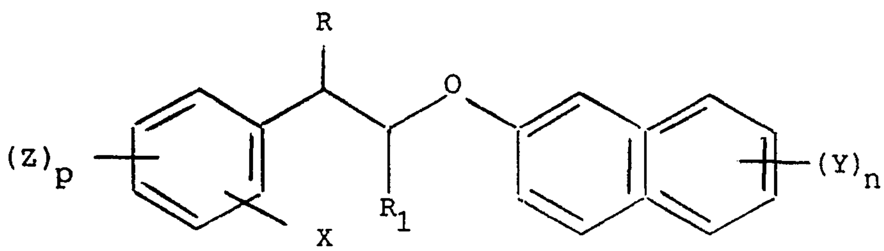

$(I_B)$

dans laquelle la définition des substituants Y, Z, R, $R_1$, n et p demeure inchangée

- les composés répondant à la formule générale $I_C$

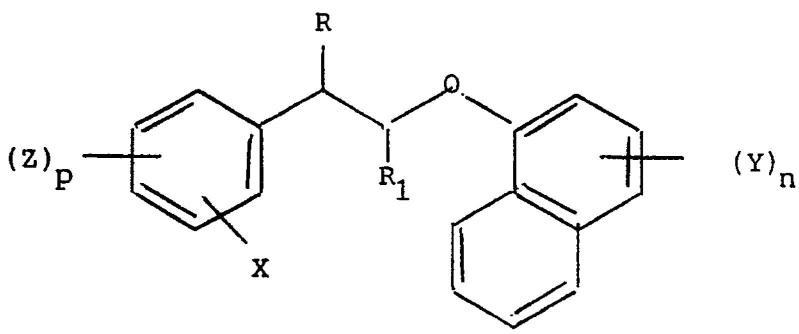

$(I_C)$

dans laquelle la définition des substituants Z, Y, R, $R_1$, X, n et p demeure inchangée

- les composés répondant à la formule générale $I_D$

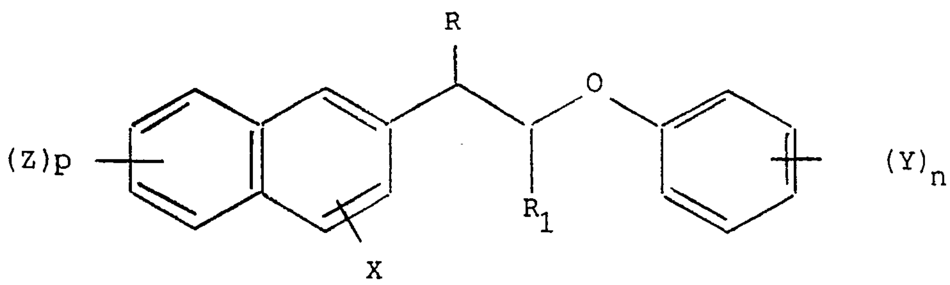

($I_D$)

dans laquelle Z, Y, X, R, $R_1$, n et p sont définis comme précédemment
- Les composés répondant à la formule générale $I_E$

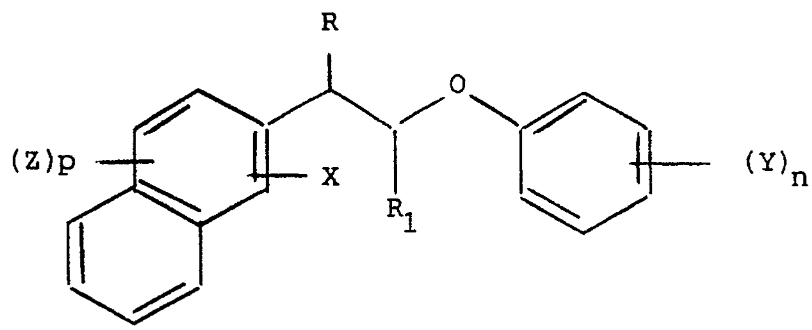

($I_E$)

dans laquelle R, $R_1$, X, Y, Z, n et p ont les significations fournies précédemment.

Parmi les composés de formule générale I, on citera tout particulièrement :
- le 1-(2-hydroxy 5-méthoxyphényl) 2-phénoxy éthane
- les phénates métalliques ou organiques du 1-(2-hydroxy 5-méthoxy phényl) 2-phénoxy éthane
- le 1-(2-hydroxy 5-carboxamido phényl) 2-(4-fluorophénoxy) éthane

Pour autant que l'invention soit concernée, le terme alcoyle inférieur désigne un radical hydrocarboné ayant de 1 à 6 atomes de carbone en chaine linéaire ou ramifiée. Des exemples de ceux-ci sont butyle, ter butyle, néopentyle, n-héxyle, méthyle ou éthyle. Un radical alcoxy inférieur comporte un radical alcoyle défini comme précédemment.

Parmi les halogènes, on citera plus particulièrement le fluor ou le chlore. Néanmoins, les dérivés bromés et iodés sont d'un intérêt équivalent.

Un radical alcenyle inférieur est un radical hydrocarboné portant une double liaison, comportant de 2 à 6 atomes de carbone. On pourra citer, à titre d'exemple de radicaux alcenyle, le radical allyle, le radical méthallyle, le radical but 2-ényle, le radical isopropényle ou le radical méthyl-3 butényle.

Un radical alcoylène dioxy comporte de 1 à 4 atomes dans la chaîne alcoylène comme, par exemple, le radical méthylène-dioxy ou éthylènedioxy.

Lorsque X et/ou Y et/ou Z représentent un radical acyle, celui-ci peut dériver d'un acide aliphatique comme un radical acétyle, propionyle, dipropylacétyle, succinoyle ou d'un acide aromatique, comme un radical benzoyle, naphtoyl-1, 2,6-dichloro benzoyle, 3,4,5-triméthoxy benzoyle, veratroyle, syringoyle, O-carbethoxy syringoyle, acétyl salicyloyl nicotinoyle, furoyle, ou d'un acide aryloxy alcanoïque comme l'acide phénoxy acétique, l'acide dichlorophénoxy acétique, l'acide p-chlorophénoxy isobutyrique ou l'acide (thenoyl-2) 2,3-dichlorophénoxy acétique.

Un radical phénylcoyle inférieur est un radical arylmonocyclique porteur d'une chaine hydrocarbonée ayant de 1 à 6 atomes de carbone, en chaine droite ou ramifiée. Des exemples de tels radicaux sont le radical benzyle, phényl-ethyle, α-méthyl phényl éthyle, 2,6-dichlorobenzyle ou 2,3,5-trimethoxy benzyle.

Lorsque Z ou Y représente un alcoxy carbonyle, il s'agit de préférence un éthoxy carbonyle, d'un méthoxy carbonyle, d'un isopropoxy carbonyle ou d'un terbutoxy carbonyle.

Z peut être également une chaine alcoyl-carboxylique dans laquelle le radical alcoyle possède de 1 à 4 atomes de carbone ainsi que les esters d'alcoyle de ceux-ci comme par exemple un méthoxy carbonyl méthyle, un éthoxy carbonyl méthyle ou un éthoxy carbonyl éthyle.

Le substituant Y peut également représenter une chaine alcoyle inférieur, substituée par un groupe oxo ou un radical hydroxyle. Un exemple de tels substituants est particulièrement le radical acétyle ou hydroxy

éthyle.

Lorsque R et/ou $R_1$ sont un alcoyle inférieur, ils sont de préférence un méthyle ou un éthyle.

De tous les composés de formule générale I, les composés actuellement préférés sont ceux pour lesquels X est un hydroxyle libre, estérifié ou éthérifié en position ortho par rapport à la chaine éthyloxy et ceux pour lesquels Y est un atome d'halogène et tout particulièrement ceux pour lesquels Y est un fluor.

La signification de Z peut varier dans des proportions importantes. La nature de ce substituant comme le nombre de substituants Z joue un rôle moindre.

L'invention s'étend aussi à un procédé d'obtention des composés de formule générale I qui consiste en ce que l'on condense un phénol de formule générale II

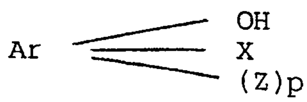

(II)

dans laquelle Ar, X, Z et p sont définis comme précédemment
avec un aryl acétonitrile de formule générale III

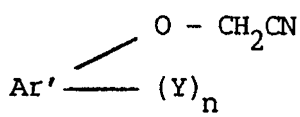

(III)

dans laquelle Y, Ar′ et n sont définis comme précédemment
en présence d'un catalyseur acide pour former une diaryl éthanone de formule générale IV

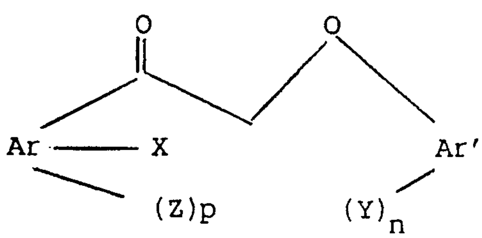

(IV)

dans laquelle Z, Y, Ar, Ar′, n et p sont définis comme précédemment
et X est un hydroxyle libre
que l'on réduit enduite par action d'un hydrure mixte de métal alcalin en présence d'un catalyseur métallique ou par réaction avec un chloroformiate d'alcoyle inférieur puis réduction du produit formé intermédiairement, par un borohydrure de métal alcalin pour obtenir un composé de formule générale I.

L'invention concerne aussi un procédé d'obtention des composés de formule $I_A$

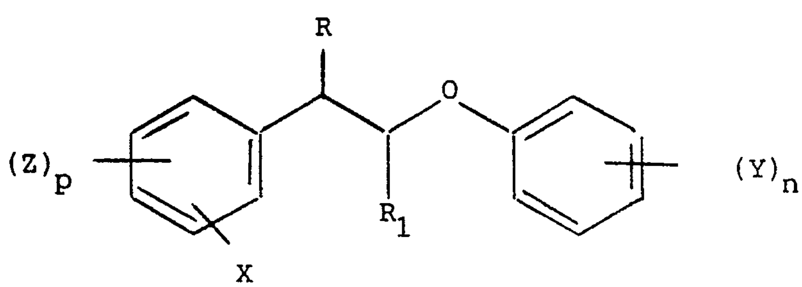

$(I_A)$

dans laquelle X représente un hydroxyle, un acyloxy dont le reste acyle dérive d'un acide carboxylique, sulfonique, phosphonique, de l'acide sulfurique et de l'acide phosphorique, un alcoxy inférieur ou un alcenyloxy inférieur

Y représente de l'hydrogène, un halogène, un alcoylène dioxy, un hydroxy, un alcoxy, un acyloxy dérivé d'un acide organique substitué par un alcoxy carbonyle, un oxo alcoyle ayant au moins deux atomes de carbone, un hydroxy alcoyle ou la chaine alcoylidène d'un cycle aromatique ayant 5 ou 6 chainons
Z représente de l'hydrogène, un hydroxyle, un alcoxy, un alcényloxy, un phénoxy, un phénylalcoyle inférieur, un alcoxy carbonyloxy, la chaine alcoylidène d'un homocycle carboné saturé ou aromatique ayant 5 ou 6 chainons, un alcoyle inférieur, un alcoylène dioxy, un acyloxy, un halogène ou un amino carbonyle
n est égal à 1, 2 ou 3
p est égal à 0, 1 ou 2
R est un hydrogène, un hydroxyle, l'oxygène d'une fonction cetone ou un alcoyle inférieur
$R_1$ est de l'hydrogène ou un radical alcoyle inférieur
qui consiste en ce que l'on condense un phénol de formule générale $II_A$

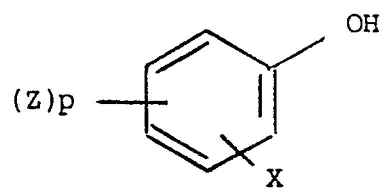

($II_A$)

dans laquelle X, Z et p sont définis comme précédemment
avec un phénoxy acéto-nitrile de formule générale $III_A$

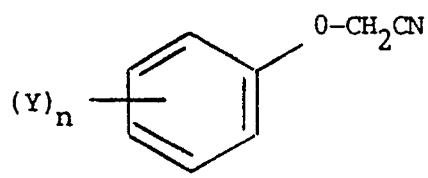

($III_A$)

dans laquelle Y et n ont les définitions antérieures en présence d'u catalyseur acide, pour former l'éthanone de formule générale $IV_A$

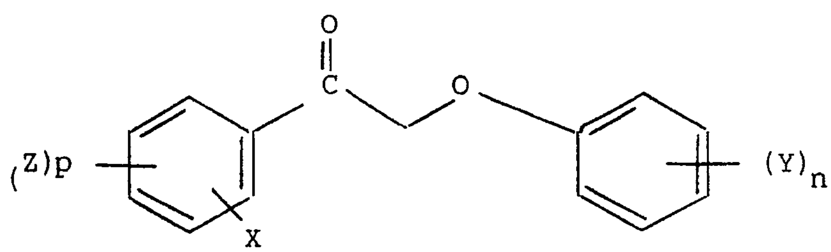

($IV_A$)

dans laquelle Z, Y, n et p ont les définitions antérieures
et X est un hydroxyle
que l'on réduit ensuite en composé de formule générale $I_A$ par action d'un hydrure mixte de métal alcalin en présence d'un catalyseur métallique ou par réaction avec un chloroformiate d'alcoyle inférieur puis réduction du produit intermédiaire par un borohydrure de métal alcalin
que l'on peut alcoyler par action d'un réactif d'alcoylation en milieu basique ou acyler par action d'un dérivé fonctionnel d'acide carboxylique, sulfonique, sulfurique ou phosphorique.

Le composé hydroxylé ainsi obtenu peut être alcoylé par action d'un réactif d'alcoylation en milieu basique ou acylé par action d'un dérivé fonctionnel d'acide carboxylique ou sulfonique comme un chlorure, un anhydride ou un anhydride mixte.

Dans ce procédé, le catalyseur acide est un acide de Lewis comme le trifluorure de bore ou le chlorure d'aluminium. On opère dans un solvant inerte comme un solvant halogéné. Un solvant approprié est par exemple le dichloroéthane.

La réduction du composé cétonique IV peut s'effectuer par les méthodes classiques de la chimie

(réaction de Clemmensen, réaction de Wolf-Kishner). Elle s'effectue au mieux par réaction avec un chloroformiate d'alcoyle puis réduction directe par un borohydrure de métal alcalin.

L'acylation de la fonction phénol s'effectue de préférence en utilisant un chlorure d'acide en présence d'une base organique comme la triethylamine.

L'alcoylation de la fonction phénol s'effectue dans l'acétonitrile comme solvant inerte. L'agent d'alcoylation est de préférence un sulfate ou un halogénure d'alcoyle.

La sulfatation de la fonction phénol s'effectue en utilisant le complexe anhydride sulfurique-triethylamine ou anhydride sulfurique-pyridine. On opère en présence d'un agent basique comme par exemple un carbonate ou un hydroxyde de métal alcalin. La phosphatation du phénol s'effectue en général en deux étapes ; chlorophosphorylation par un oxychlorure puis hydrolyse du composé phosphorodichloridate qui s'est formé à titre intermédiaire. Les dérivés sulfuriques ou phosphoriques sont aisément salifiés par un agent basique.

L'invention concerne aussi un procédé de préparation des composés de formule générale I caractérisé en ce qu on bloque la fonction phénol d'une hydroxy-acétophénone de formule générale V

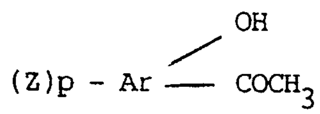

(V)

dans laquelle Ar, Z et p sont définis comme précédemment
et X est un hydroxyle libre
par un réactif aisément clivable, puis soumet le phénol bloqué de formule VI

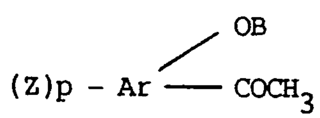

(VI)

dans laquelle B est un radical aisément clivable
Z et p sont définis comme précédemment
que l'on soumet à l'action d'un agent de bromation pour former une α-bromocétone de formule VII

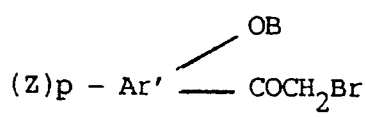

(VII)

dans laquelle Z, AR et p ont les mêmes significations que précédemment
condense celle-ci avec un phénol de formule

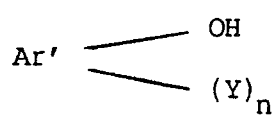

dans laquelle Ar′, Y et n sont définis comme précédemment
pour obtenir une phénoxy éthanone de formule générale VIII

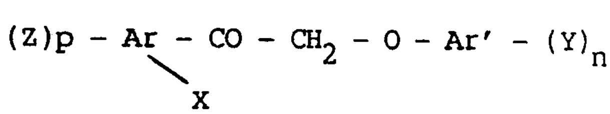

(VIII)

dans laquelle la définition des substituants Y, Z, Ar, n et p demeure inchangée
et X est un radical hydroxy bloqué par une fonction aisément clivable
puis soumet le composé VIII à une hydrogénation en présence d'un catalyseur métallique ou à une acidolyse, pour libérer la fonction phénol en formant une phénoxy éthanone de formule générale IX

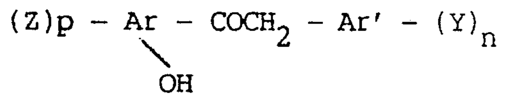

(IX)

dans laquelle Y, Z, n, p, Ar et Ar′ sont définis comme précédemment
traite cette dernière par un agent réducteur pour former l'alcool correspondant de formule générale X

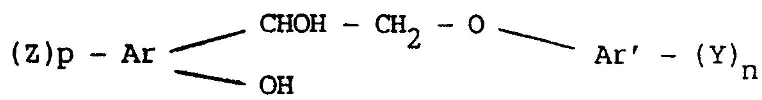

(X)

dans laquelle Z, Y, n et p sont définis comme précédemment et X est un hydroxyle libre.

L'invention a encore pour objet un procédé d'obtention des composés de formule générale $I_A$

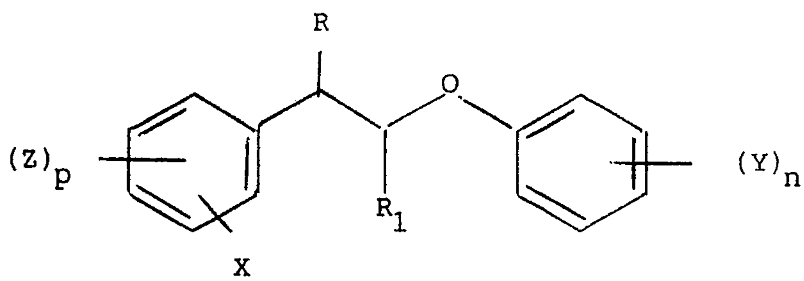

($I_A$)

dans laquelle Z, X, R, $R_1$, Y, n et p sont définis comme précédemment
dans lequel on bloque la fonction phénol d'une hydroxy acétophénone de formule générale $V_A$

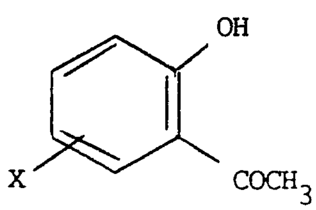

($V_A$)

par un réactif labile puis soumet à l'action d'un agent de bromation le phénol bloqué, pour former une α-bromocétone, condense la cétone α-bromée ainsi formée avec un phénol pour former une phénoxyéthanone de formule $VI_A$

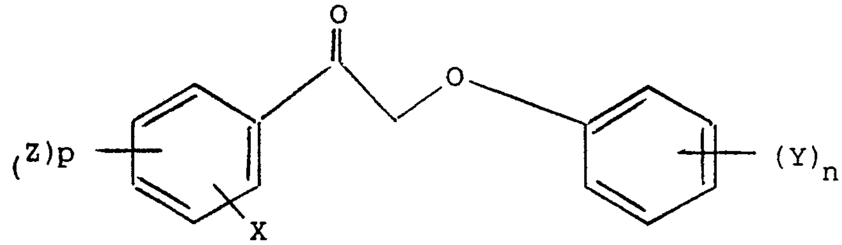

($VI_A$)

dans laquelle les substituants Y, Z, n et p ont les significations fournies antérieurement

et X est une fonction phénol bloquée
puis soumet le composé $VI_A$ à une hydrogénation en présence d'un catalyseur métallique ou à une acidr la fonction phénol en formant une phénoxyéthanone de formule générale $VII_A$

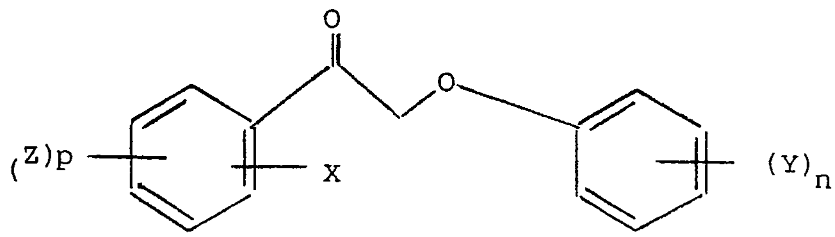

($VII_A$)

dans laquelle Y, Z, n et p sont définis comme précédemment
et X est un hydroxyle
puis le composé de formule $VII_A$ est soumis à l'action d'un agent réducteur pour former l'alcool correspondant de formule générale $VIII_A$

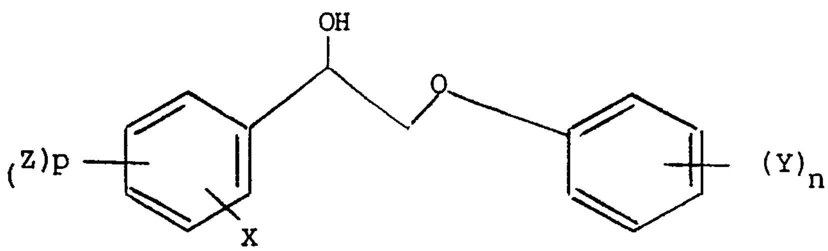

($VIII_A$)

dans laquelle Z, Y, n et p sont définis comme précédemment
et X est un hydroxyle libre

Dans ce procédé, la réaction de bromation de l'acétophénone est effectuée par un agent de bromation comme par exemple le brome en présence de chlorure d'Aluminium dans l'ether ou le complexe brome triethylamine dans le tétrachlorure de carbone ou bien encore, le tribromure de phényl-trimethyl anilinium (PTT) dans un solvant éthéré comme le tétrahydrofurane.

La condensation du dérivé α-bromé avec un phénol s'effectue en présence d'un agent alcalin comme par exemple un carbonate de métal alcalin ou alcalino-terreux, dans un solvant polaire comme par exemple l'acétonitrile.

Le dérivé VI résultant est hydrogénolysé par l'hydrogène en présence d'un catalyseur métallique comme le platine ou le palladium, éventuellement déposé sur un support comme le charbon ou le carbonate de Calcium. Le dérivé VI peut également être soumis à une acidolyse par reflux d'un mélange d'acide chlorhydrique et d'acide acétique.

Les intermédiaires cétoniques et notamment les composés de formule générale VII sont réduits en alcool par action d'un hydrure mixte de métal alcalin comme le borohydrure de sodium ou le borohydrure de potassium en opérant dans un alcanol comme par exemple le méthanol ou l'éthanol.

L'invention s'étend encore à un procédé d'obtention des composés de formule générale I dans laquelle R est un radical alcoyle inférieur, caractérisé en ce que l'on soumet une phénoxy éthanone de formule générale VIII

$$(Z)p - \underset{\diagdown X}{Ar} - CO - CH_2O - Ar' - (Y)_n \qquad (VIII)$$

dans laquelle Z, Y, Ar, Ar', p et n sont définis comme précédemment
et X est un hydroxyle bloqué
à l'action d'un bromure de triarylalcoyl phosphonium dans les conditions de la réaction de WITTIG pour former un dérivé éthylénique de formule générale IX

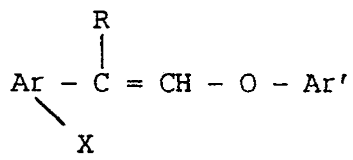

(IX)

dans laquelle Z, Y, Ar, Ar′, n et p sont définis comme précédemment
X est une fonction phénol bloquée
et R est un radical alcoyle inférieur
puis soumet ce dernier composé à une hydrogénation en présence d'un catalyseur métallique pour obtenir le composé α-alcoylé de formule générale I

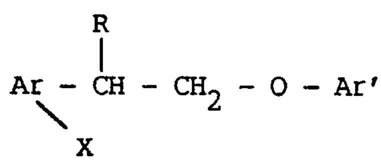

(I)

dans laquelle Z, Y, Ar, Ar′, n et p sont définis comme précédemment
X est un hydroxyle libre
et R est un radical alcoyle inférieur
que l'on peut, si désiré, alcoyler ou acyler dans les conditions définies précédemment

La réaction de Wittig sur le composé VIII est effectuée de préférence en présence d'un alcoolate de métal alcalin comme le teramylate de potassium ou le terbutylate de sodium ou bien encore, en présence d'un alcoyl lithium comme le butyl-lithium. Le dérivé éthylénique de formule IX est hydrogéné en dérivé saturé de formule générale I par l'hydrogène en présence d'un métal noble comme par exemple le palladium.

L'invention concerne aussi un procédé de préparation des composés de formule générale $I_A$ dans laquelle R est un radical alcoyle inférieur dans lequel un composé de formule générale

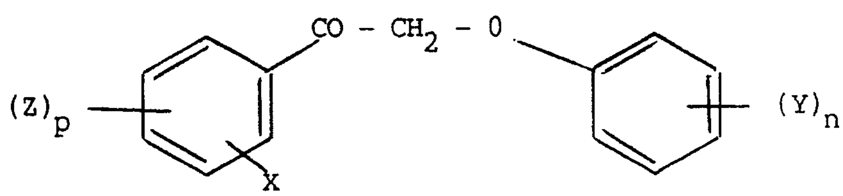

$(VI_A)$

dans laquelle les substituants Y, Z, n et p ont les significations fournies antérieurement
et X est une fonction phénol bloquée
est soumis à l'action d'un bromure de triaryl alcoyl phosphonium dans les conditions de la réaction de Wittig pour former un dérivé éthylénique de formule générale $IX_A$

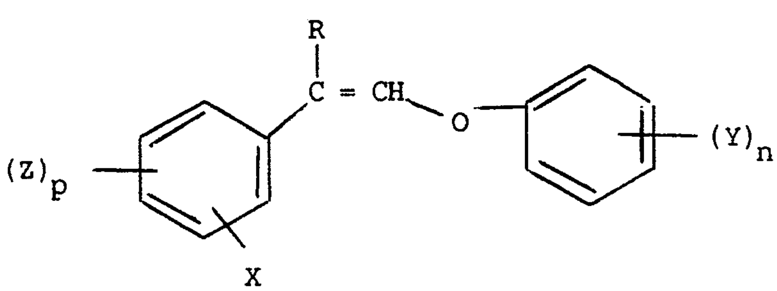

$(IX_A)$

dans laquelle Z, Y, n et p ont les significations antérieures

X est une fonction phénol bloquée
et R est un radical alcoyle inférieur
puis soumet ce dernier composé à une hydrogénation en présence d'un catalyseur métallique et obtient un composé $\alpha$-alcoylé de formule générale $I_A$

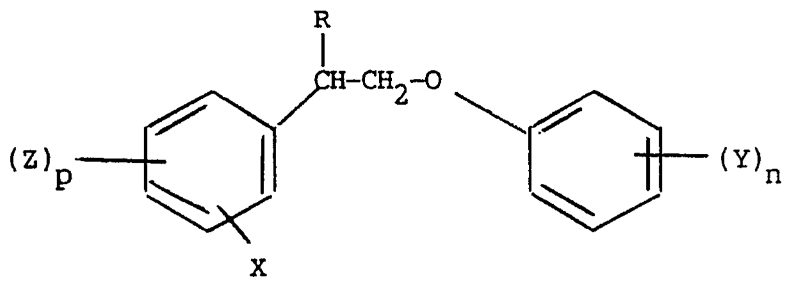

($I_A$)

dans laquelle Z, Y, n et p sont définis comme précédemment
X est un hydroxy
et R est un radical alcoyle inférieur
que l'on peut, si désiré, alcoyler ou acyler dans les conditions définies précédemment.

L'invention s'étend également à un procédé d'obtention des composés de formule générale I caractérisé en ce que l'on soumet un benzaldéhyde substitué de formule générale XI

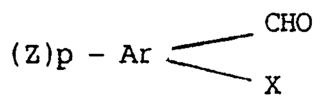

(XI)

dans laquelle Z, p et Ar ont les significations fournies antérieurement
et X est une fonction phénol bloquée
à l'action d'un sel de triaryl phénoxy méthyl phosphonium de formule

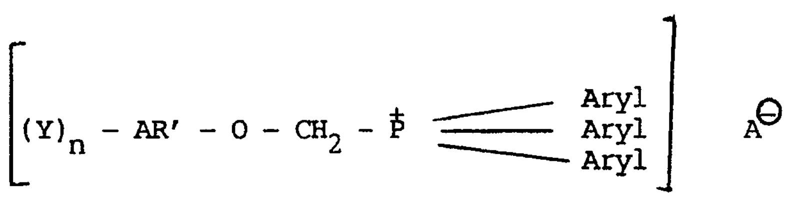

dans laquelle Y, Ar′ et n sont définis comme précédemment
et A est un anion dérivé d'un acide général non réactif dans les conditions de la réaction de WITTIG, pour former un dérivé éthylénique de formule générale XII

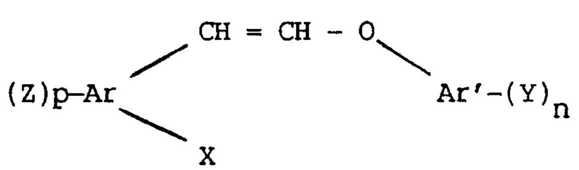

(XII)

dans laquelle Z, Ar, X, Ar′, Y, n et p ont les significations fournies précédemment
que l'on hydrogène par l'hydrogène en présence d'un catalyseur métallique en composé de formule générale I

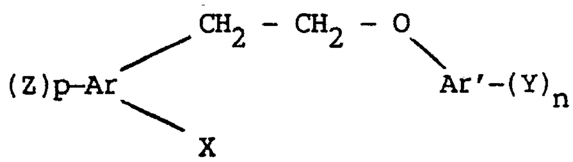

(I)

dans laquelle les substituants Z, Ar, Ar′, Y, n, p et X ont les significations antérieures

L'invention comprend également un procédé d'obtention des composés de formule générale $I_A$ dans lequel on soumet un benzaldéhyde substitué de formule générale $X_A$

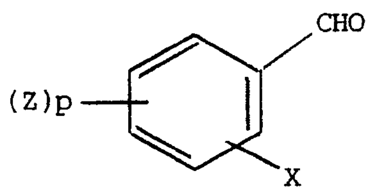

($X_A$)

dans laquelle Z et p ont les définitions antérieures
et X est une fonction phénol bloquée
à l'action d'un sel de triarylphénoxy méthyl phosphonium dans les conditions de la réaction de WITTIG pour former un dérivé éthylénique de formule $XI_A$

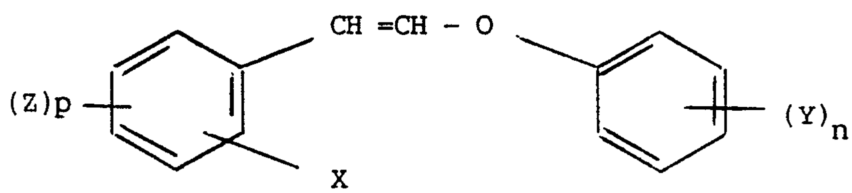

($XI_A$)

dans laquelle Z, Y, n et p ont les significations fournies antérieurement
et X est une fonction phénol bloquée
que l'on hydrogène en composé de formule générale $I_A$ par l'hydrogène en présence d'un catalyseur métallique

Dans ce procédé lorsque Z représente un substituant sensible à l'hydrogénation comme par exemple un substituant benzyloxy, l'hydrogénolyse entraine en même temps, la réduction ou la libération de la fonction concernée.

L'invention comprend aussi un autre procédé d'obtention des composés de formule générale I caractérisé en ce qu'on bloque les fonctions réactives d'un hydroxyaryl éthanol de formule générale XIII

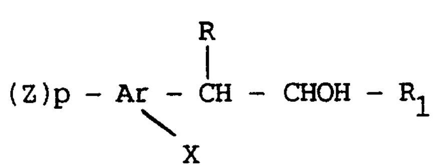

(XIII)

dans laquelle Z, Ar, Ar′, R et $R_1$ sont définis comme précédemment
et X est un hydroxyle libre
à l'aide d'un réactif aisément clivable, puis fait réagir le dérivé ainsi bloqué avec un phénol de formule générale XIV

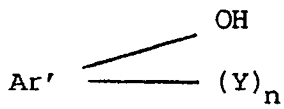

(XIV)

dans laquelle Y, n et Ar′ ont les significations fournies antérieurement
pour fournir un phénoxyéthyl benzène de formule générale XV

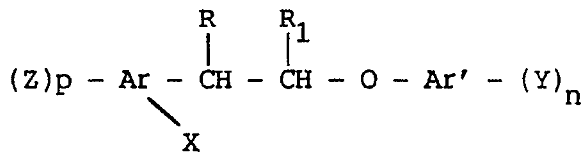

(XV)

dans laquelle Z, Y, Ar, Ar′, n et p sont définis comme précédemment
R et $R_1$, distinctement l'un de l'autre, sont de l'hydrogène ou un radical alcoyle inférieur
et X est un hydroxyle bloqué
dont on libère la fonction hydroxyle par action d'un agent alcalin pour former un composé de formule générale I.

L'invention concerne aussi un procédé d'obtention des composés de formule générale $I_A$ dans lequel on bloque les fonctions réactives d'un hydroxy phényl éthanol de formule générale $XII_A$

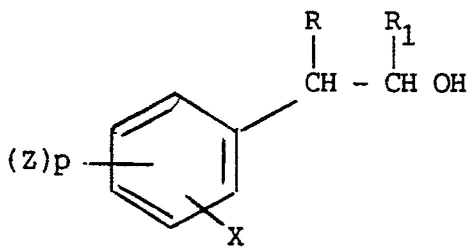

($XII_A$)

dans laquelle Z et p sont définis comme précédemment
X est un hydroxyle
R et $R_1$ sont de l'hydrogène ou un radical alcoyle inférieur
à l'aide d'un réactif aisément clivable, fait réagir le dérivé bloqué avec un phénol de formule générale $XIII_A$

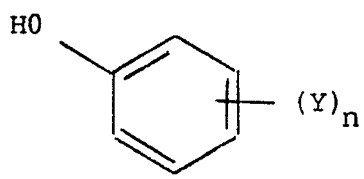

($XIII_A$)

dans laquelle Y et n ont les définitions antérieures
pour obtenir un phényléthoxy phényle de formule générale $XIV_A$

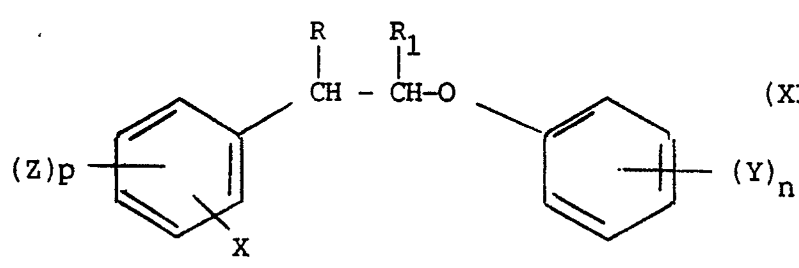

($XIV_A$)

dans laquelle Z, Y, n et p sont définis comme précédemment
R et $R_1$ sont de l'hydrogène ou un radical alcoyle inférieur
et X est un hydroxyle bloqué
puis libère la fonction hydroxyle par action d'un agent alcalin pour former un composé de formule générale $I_A$

Dans ce procédé, le groupe protecteur est de préférence un dérivé fonctionnel d'acide alcoyl sulfonique ou d'acide arylsulfonique comme l'acide méthane sulfonique et la libération des fonctions bloquées s'effectue par action d'un hydroxyde de métal alcalin en milieu aqueux.

L'invention concerne aussi, à titre de produits nouveaux, les produits intermédiaires formés au cours de la synthèse et notamment les dérivés éthyléniques de formule générale $IX_A$

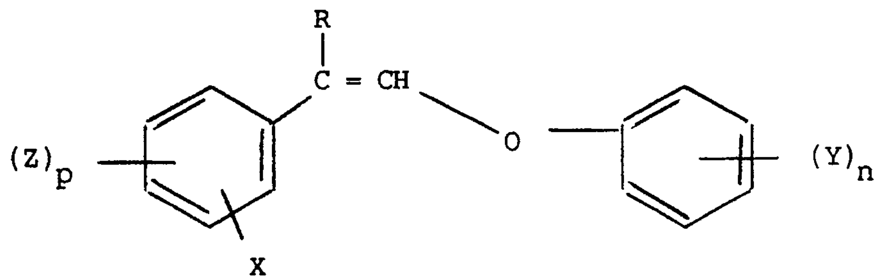

$(IX_A)$

dans laquelle Z, Y, X, R, n et p ont les significations fournies précédemment
et les dérivés éthyléniques de formule générale $XII_A$

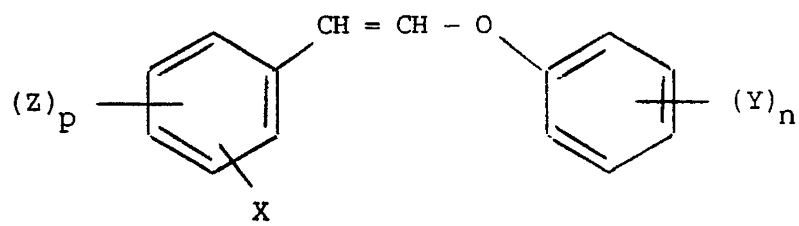

$(XII_A)$

dans laquelle Z, Y, X, p et n sont définis comme précédemment.

Les composés de formule générale I possèdent des propriétés pharmacologiques très intéressantes et notamment des propriétés diurétiques, anti-hypertensives, anti-agrégantes plaquettaires et anti-lipoxygénase.

Ces propriétés vasculaires sont originales car leur site d'action n'est pas exclusivement rénal. Les composés selon l'invention inhibent la contraction induite par la noradrénaline et par le potassium.

Ces résultats impliquent l'existence d'un site d'action extra-rénal probablement vasculaire dans le mécanisme d'action anti-hypertensive des composés de formule générale I. Les résultats pharmacologiques obtenus permettent de penser que l'action des composés de formule générale I passe par le mouvement intracellulaire du Calcium dans la paroi musculaire des vaisseaux.

En raison de leurs propriétés pharmacologiques, les composés selon l'invention trouvent un emploi en thérapeutique comme médicament anti-agrégant plaquettaire ou comme médicament anti-hypertenseur.

A ces fins, ils sont employés sous forme de compositions pharmaceutiques qui renferment à titre de principe actif au moins un composé de formule générale I en association ou en mélange avec un excipient ou un véhicule inerte, non toxique, pharmaceutiquement acceptable.

Pour l'usage thérapeutique, ils seront présentés sous une des formes pharmaceutiques appropriées pour l'administration par voie parentérale, digestive, rectale, permuqueuse ou transcutanée.

On pourra citer à cet égard, les solutés ou suspensions injectables présentés en ampoules, en flacons multi-doses ou en seringues auto-injectables, les comprimés nus ou enrobés, les dragées, les gélules, les poudres, les suppositoires, les solutés dans un solvant polaire ou bien encore les préparations à usage transdermique.

Les excipients qui conviennent pour de telles administrations sont les dérivés de la cellulose ou la cellulose micro-cristalline, les carbonates alcalino-terreux, le phosphate de magnésium, les amidons, les

amidons modifiés ou le lactose.

Pour l'usage rectal, le beurre de cacao ou les stérrates de polyéthylène glycol sont les excipients préférés.

Pour l'usage parentéral, l'eau, les solutés aqueux, le sérum physiologique, le soluté de glucose sont les véhicules les plus commodément utilisés.

La posologie peut varier dans des limites importantes en fonction de la sévérité de la maladie hypertensive, de l'âge et du poids du sujet et de la voie d'administration.

En règle générale, la posologie unitaire pourra s'échelonner de 1 à 200 mg par prise et la posologie journalière pourra s'échelonner de 2 à 500 mg.

L'administration sera modulée de préférence en raison de la durée d'action des composés de formule générale I.

Les exemples suivants illustrent l'invention. Ils ne la limitent en aucune façon :

## EXEMPLE I

### 1-(2-hydroxy 5-méthoxy phényl) 2-phénoxy éthane

(procédé de synthèse A)

Stade A : 1-(2-hydroxy 5-méthoxy phényl) 2-phénoxy éthanone

A une solution de 32,2 g (0.27 mole) de trichlorure de bore dans le dichlorethane (200 ml) mise à 0°, on ajoute successivement une solution formée de 28,4 g (0.23 mole) de 4-méthoxy phénol dans 100 ml de dichlorethane, 36,6 g (0.24 mole) de phénoxy acétonitrile puis 15,2 g (0.11 mole) de chlorure d'aluminium sous agitation. Le mélange est maintenu sous agitation pendant 20 heures à température ordinaire. Après hydrolyse en milieu acide pendant 30 mn et extraction, on obtient après cristallisation dans le méthanol 26 g (soit un rendement de 44%) de phénoxy acétophénone. Le produit pur fond à 128° C.

Stade B : 1-(2-hydroxy 5-méthoxy phényl) 2-phénoxy éthane

On ajoute à 0° C 6,4 g (0.058 mole) de chloroformiate d'éthyle à une solution de 12,8 g de 1-(2-hydroxy 5-méthoxy phényl) 2-phénoxy éthanone obtenue au stade A dans 100 ml de tétrahydrofuran en présence de 6 g (0.058 mol) de triéthylamine. Après une heure de contact sous agitation à température ordinaire, on filtre le chlorhydrate de triethylamine formé et on introduit le filtrat goutte à goutte dans une solution de 5,6 g (0.15 mol) de borohydrure de sodium dans 50 ml d'eau à 5° C pendant 45 mn. Après agitation à température ordinaire pendant 1 h 30, on verse le milieu dans la glace, on soumet à une hydrolyse acide avec de l'acide chlorhydrique puis on extrait à l'éther. Après distillation du solvant, on obtient 8,4 g de produit purifié par distillation sous pression réduite (rendement 69 %) PF = 170° C

## EXEMPLE II

### 1-(2-méthoxy phényl) 2-phénoxy éthane

4 g de 1-(2-hydroxy phényl) 2-phénoxy éthane (0.032 mole) et 16 g de carbonate de potassium (0.117 mole) dans 100 ml d'acétonitrile sont additionnés de 4,9 g (0.039 mole) de sulfate de méthyle et portés au relfux pendant 5 heures. Après évaporation et extraction, on isole 5,6 g (Rendement 80 %) de dérivé 2-méthoxylé sous forme d'un produit huileux.

## EXEMPLE III

**1-(2-acétoxy 5-méthoxy phényl) 2-phénoxy éthane**

On ajoute à 4 g (0.016 mole) de 1-(2-hydroxy 5-méthoxy phényl) 2-phénoxy éthane 1,5 g (0.019 mole) de chlorure d'acétyle et 2 g (0.019 mole) de triéthylamine en vue de l'acétylation. Après agitation 1 heure à température ordinaire, le milieu est extrait pour fournir 4 g soit 85 % de dérivé 2-acétoxylé.

**EXEMPLE IV**

**Sel de potassium du 1-(2-sulfitoxy phényl) 2-(4-fluorophénoxy) éthane**

Dans un ballon à trois tubulures sous atmosphère d'argon, on introduit 6,53 g (0.47 mole) de carbonate de potassium et 10 g (0.43 mole) de 1-(2-hydroxyphényl) 2-(4-fluorophénoxy) éthane dans 100 ml d'acétonitrile. On agite pendant 30 mn à 40° C puis on ajoute 8,97 g (0.645 mole) du complexe [$SO_3$ trimethylamine].Après agitation, le mélange est refroidi et le sulfate formé précipité.

Après filtration, on isole 6,5 g de solide blanc.

F = 163° C Rendement = 42 %

**EXEMPLE V**

**1-(2-phosphonyloxy phényl) 2-(4-fluorophénoxy) éthane**

Stade A : 2-[(4-fluorophénoxy) éthyl] phényl phosphorodichloridate

On dissout 10 g (0.043 mole) de 1-(2-hydorxyphényl) 2-(4-fluorophénoxy) éthane dans 36 g de $POCl_3$ - (0.235 mole) et on porte au reflux pendant 2 heures 30 en présence de 0,1 g de chlorure d'aluminium. Le milieu est concentré puis distillé. Le phosphate formé distille Eb 0,25 = 210 - 220° C. On recueille 9,71 g d'un produit huileux (rendement = 64 %)

Stade B : Hydrolyse du phosphorodichloridate

On hydrolyse 4 g (0.11 mole) de phosphorodichloridate dans 10 ml d'eau sous agitation à 80° C. Le mélange est maintenu sous agitation pendant 3 heures. On concentre ensuite sous vide jusqu'à siccité. Par trituration dans l'éther de pétrole, on recueille un solide blanc pesant 3,1 g.

(Rendement = 86 %) F = 80-82° C

**EXEMPLE VI**

**1-(2 hydroxyphényl) 2-(4-fluorophénoxy) éthanol** (procédé B)

Stade A : 1-(2-benzyloxy phényl) 2-bromoéthanone

On dissout 250 g (1.1 mole) de 2-benzyloxyacétophénone dans 1 l de tétrahydrofuran sec. On refroidit à 0° et on introduit alors par petites quantités 500 g (1,32 mole) de tribromure phényl triméthyl anilinium, pendant 4 heures. On filtre le précipité. On évapore le mélange à sec et le résidu est utilisé tel quel pour la suite de la synthèse.

Poids : 34 g environ

Rendement : 70%

Stade B : 1-(2-benzyloxyphényl) 2-phénoxy ethanone

125 g du dérivé α-bromé précédent sont additionnés de 57 g de phénol (0.61 mole) et de 283 g (2 moles) de carbonate de potassium dans l'acétonitrile et on porte au reflux pendant 2 heures. Après filtration des sels minéraux et évaporation du solvant, on reprend par le dichloréthane, lave avec une solution d'hydroxyde de sodium N puis à l'eau jusqu'à neutralité des eaux de lavage. On sèche ensuite puis évapore le solvant pour fournir 104 g de dérivé phénoxybenzylé.
F = 141 °C Rendement = 80 %

Stade C : 1-(2-hydroxy phényl) 2-phénoxy éthane

52 g (0.164 mole) de dérivé phénoxybenzylé du stade B sont mis à hydrogéner sous atmosphère d'hydrogène dans l'éthanol (1.000 ml) en présence de 3 g de charbon palladié à 10 %. Après absorption de l'hydrogène, on sépare le catalyseur par filtration, on évapore l'éthanol et le résidu sec est cristallisé pour fournir 34,7 g de dérivé phénolique. Le rendement est de 92% - F = 120 °C

Stade D : 1-(2-hydroxyphényl) 2-4-fluorophénoxy) éthanol

A une solution de 5 g (0.02 mole) de 1-(2-hydroxy phényl) 2-(4-fluorophénoxy) éthanone dans le méthanol refroidi à 0 °C, on ajoute peu à peu 0,760 g (0.02 mole) de borohydrure de sodium. Après retour à la température ordinaire, au bout d'une heure, on concentre le milieu réactionnel on le jette sur de la glace puis acidifie à pH4 et extrait à l'éther pour obtenir 4,5 g du dérivé hydroxy éthylé sous forme d'un produit huileux.
Rendement = 90 %

**EXEMPLE VII**

**2-(2-hydroxyphényl) 3-phénoxy propane**

A une solution de 53,5 g de bromure de triphényl méthyl phosphonium dans 500 de tétrahydrofuran sous atmosphère d'argon, on ajoute 112,5 ml d'une solution 1,6 M de butyl lithium dans l'hexane à températ.ure ambiante. Après 4 heures d'agitation, on verse goutte à goutte la 1-(2-benzyloxyphényl) 2-phénoxy éthanone en solution dans le tétrahydrofuran et on porte une nuit au reflux.

Après concentration du solvant, on verse le mélange dans l'eau et on extrait à l'éther. Le résidu après évaporation du solvant est soumis à une chromatographie-éclair (flash chromatography) sur silice avec mélange d'éluants Ether de pétrole/Ether isopropylique 90:10 pour obtenir 37 g de dérivé propénique. F = 57 °C

On hydrogénolyse 24 g de dérivé propénique sous atmosphère d'hydrogène en présence de charbon palladié à 10% de Palladium dans 400 ml d,éthanol sous bonne agitation, à pression atmosphérique et à 50 °C pendant 20 heures.

Après séparation du catalyseur par filtration et évaporation du filtrat, on isole 16 g de 2-(2-hydroxyphényl) 3-phénoxy propane
Rendement = 75 %

**EXEMPLE VIII**

**1-(3-méthoxy 4-hydroxy phényl) 2-(4-fluorophénoxy) éthane**

- Préparation de l'éther (chlorométhyl) (4-fluorophénoxy)

On chauffe 59 g (0.47 mole) de 4-fluoroanisole avec 97 g (0.47 mole) de pentachlorure de phosphore à

120 °C pendant 2 heures puis la température est élevée à 140 puis 160 ° au fur et à mesure que $PCl_3$ distille. On laisse encore 2 heures à cette température après achèvement de la distillation de $PCl_3$ puis on distille sous pression réduite (15 mmHg) à la température de 100 °C pour obtenir 47 g de dérivé chloré. Rendement = 75 %

- Préparation du chlorure de triphényl (4-fluorophénoxyméthyle) phosphonium

On introduit dans 300 ml de benzène 42 g du dérivé chlorométhylé (0.26 mole) et 81 g de triphénylphosphine et on porte le mélange au reflux pendant une nuit. Après évaporation du benzène on fait cristalliser le sel de phosphonium de l'éther.

On isole 62 g de produit F = 248 °C
Le rendement est de 57 %.

Stade A : 1-(3-méthoxy 4-benzyloxyphényl) 2-(4-fluorophénoxy) éthène

On dissout 12,2 g (0.029 mole) de chlorure de (4-fluorophénoxy méthyl) triphénylphosphonium dans 100 ml de tétrahydrofuran puis on refroidit à -78 °C sous atmosphère d'argon.

On ajoute goutte à goutte 16 ml d'une solution 1,6 M (soit 0.026 mole) de butyl lithium dans l'hexane. Après 1 heure à cette température, on ajoute 5 g (0.02 mole) de 3-méthoxy 4-benzyloxy benzaldehyde puis on laisse revenir à température ordinaire en deux heures. On jette dans la glace, acidifié à pH 4 et extrait à l'éther. Après évaporation du solvant, le résidu est filtré sur silice et on isole 6,2 g (86 %) de phénoxyéthène.

Stade B : 1-(3-méthoxy 4-hydroxyphényl) 2-(4-fluorophénoxy) éthane

On place 8,2 g (0.0225 mole) de dérivé phénoxy éthénique dans un récipient à hydrogéner sous atmosphère d'hydrogène dans l'éthanol en présence de 2 g de charbon palladié à 10 %.

On maintient sous bonne agitation pendant 12 heures. Après filtration du catalyseur et évaporation du filtrat, on chromatographie le résidu sur silice en éluant par le mélange ether de pétrole/acétate d'éthyle 90:10, pour fournir après évaporation du solvant, 3,5 g de dérivé saturé sous forme d'un produit huileux. Rendement = 60 %.

**EXEMPLE IX** (méthode D)

**1-(3-hydroxyphényl) 2-(4 fluorophénoxy) éthane**

Stade A : 1-(2-méthanesulfonyloxyéthyl) 3-méthanesulfonyloxy benzène

On ajoute à 29 g (0,21 mole) de (3-hydroxyphényl) éthanol dans la pyridine anhydre, à 0 °C, 41 ml (0.525 mole) de chlorure de méthane sulfonyle goutte à goutte sous agitation .

Après retour à la température ordinaire pendant 1 H 30, le milieu réactionnel est versé sur de la glace. On acidifie puis extrait au chlorure de méthylène. On lave à l'eau jusqu'à neutralité, sèche sur sulfate de sodium et évapore à sec. On obtient 53 g de diméthane sulfonate
Rendement = 86 %

Stade B : 1-(3-méthanesulfonyloxyphényl-)2-(4-fluorophénoxy) éthane

On introduit dans 350 ml d'acétone un mélange formé de 53 g (O.18 mole) du diméthane sulfonate du stade A, 24,4 g (0.217 mole) de 4-fluorophénol et de 28,8 g (0.217 mole) de Carbonate de potassium. On porte au reflux sous agitation pendant 9 heures. On filtre ensuite les sels minéraux et évapore le filtrat. On reprend le résidu par de l'eau, et on épuise à l'éther. Les phases organiques sont séparées, lavées à la

soude puis à l'eau jusqu'à neutralité et séchées sur sulfate de sodium. On évapore à sec pour obtenir 46 g de résidu (83 %) de (3-méthyl sulfonyloxyphényl) 2-(4-fluoro phénoxy) éthane suffisamment pur pour la poursuite de la synthèse.

Stade C : 1-(3-hydroxyphényl-) 2-(4-fluorophénoxy) éthane

On effectue la saponification de 46 g (0.148 mole) de méthane sulfonate du stade B au reflux dans 1.000 ml de soude 2N pendant 16 heures. Après refroidissement, on acidifie par ClH 5N, on épuise à l'éther et on sèche sur sulfate de sodium.

On évapore et on recueille 25 g de produit brut renfermant du 3-hydroxy styrène. Par Flash chromatography et élution par un mélange éther de pétrole/éther isopropylique 80:20, on obtient 11,68 g de (3-hydroxy phényl) 2-(4-fluorophénoxy) éthane pur sous forme cristallisée F = 64 °C

Les composés suivants répondant à la formule générale I ont été préparés par l'une des méthodes de synthèse A, B, C ou D. Leurs constantes sont énumérées dans le tableau VI, ci-après.

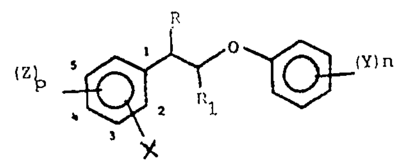

TABLEAU VI

| Ex | (Z)p | (Y)n | X | R | METHODE | FORMULE BRUTE | PM | PF°C | ANALYSE CENTESIMALE CALCULEE % C | H | N | TROUVEE % C | H | N |
|---|---|---|---|---|---|---|---|---|---|---|---|---|---|---|
| 12 | H | H | 2-0CH | H | A | $C_{14}H_{14}O_2$ | 214,267 | 45 | 78,48 | 6,59 | - | 78,42 | 6,55 | - |
| 13 | H | H | $2-OCOCH_3$ | H | A | $C_{16}H_{16}O_3$ | 256,304 | Huile | 74,98 | 6,29 | - | 74,86 | 6,21 | - |
| 14 | H | H | $2-OCH_2CH_3$ | H | A | $C_{17}H_{18}O_3$ | 270,331 | Huile | 75,53 | 6,71 | - | 75,56 | 6,68 | - |
| 15 | H | H | 2-0CO 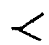 | H | A | $C_{18}H_{20}O_3$ | 284,358 | Huile | 76,03 | 7,08 | - | 76,01 | 7,13 | - |
| 16 | H | H | 2-0 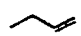 | H | A | $C_{17}H_{18}O_2$ | 254,330 | Huile | 80,28 | 7,13 | - | 80,10 | 7,02 | - |
| 17 | H | H | $2-OCH_3$ | H | A | $C_{15}H_{16}O_2$ | 228,294 | Huile | 78,91 | 7,06 | - | 78,77 | 6,97 | - |
| 18 | H | H | 2-0CO 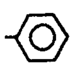 | H | A | $C_{21}H_{18}O_3$ | 318,375 | 72 | 79,22 | 5,69 | - | 79,28 | 5,66 | - |
| 19 | H | H | 2-0CO 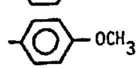 | H | A | $C_{22}H_{20}O_4$ | 348,40 | 48 | 75,84 | 5,78 | - | 75,85 | 5,72 | - |
| 20 | H | H | 2-0CO 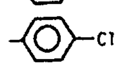 | H | A | $C_{21}H_{17}ClO_3$ | 352,817 | 56 | 71,49 | 4,88 | - | 71,26 | 4,69 | - |
| 21 | H | H | 2-0CO 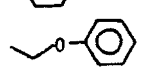 | H | A | $C_{22}H_{20}O_4$ | 348,402 | 54 | 75,84 | 5,78 | - | 75,65 | 5,82 | - |
| 22 | H | H | 2-0CO 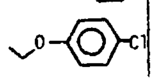 | H | A | $C_{22}H_{19}ClO_4$ | 382,844 | 70 | 69,02 | 5,00 | - | 69,10 | 5,10 | - |

| EX | (Z)p | (Y)n | X | R | METHODE | FORMULE BRUTE | PM | PF°C | ANALYSE CENTESIMALE CALCULEE % C | H | N | TROUVEE % C | H | N |
|---|---|---|---|---|---|---|---|---|---|---|---|---|---|---|
| 23 | H | H | $2\text{-}OSO_2CH_3$ | H | A | $C_{15}H_{16}O_4S$ | 292,353 | 68 | 61,62 | 5,51 | - | 61,85 | 5,68 | - |
| 24 | $5\text{-}OCH_3$ | 4-F | 2-OH | H | A | $C_{15}H_{15}FO_3$ | 262,285 | 60 | 68,69 | 5,76 | - | 68,56 | 5,70 | - |
| 25 | $5\text{-}OCH_3$ | 4-F | $2\text{-}OCOCH_3$ | H | A | $C_{17}H_{17}FO_4$ | 304,323 | Huile | 67,09 | 5,63 | - | 67,03 | 5,59 | - |
| 26 | $5\text{-}OCH_3$ | 4-Cl | 2-OH | H | A | $C_{15}H_{15}ClO_3$ | 278,735 | 86 | 64,64 | 5,42 | - | 64,49 | 5,50 | - |
| 27 | $5\text{-}OCH_3$ | 4-Cl | $2\text{-}OCOCH_3$ | H | A | $C_{17}H_{17}ClO_4$ | 320,773 | Huile | 63,65 | 5,34 | - | 63,41 | 5,18 | - |
| 28 | $5\text{-}OCH_3$ | H | 2-OH | H | A | $C_{15}H_{16}O_3$ | 244,293 | 59 | 73,75 | 6,60 | - | 73,63 | 6,69 | - |
| 29 | $5\text{-}OCH_3$ | H | $2\text{-}OCOCH_3$ | H | A | $C_{17}H_{18}O_4$ | 286,331 | Huile | 71,31 | 6,33 | - | 71,40 | 6,35 | - |
| 30 | H | 4-F | 2-OH | H | A | $C_{14}H_{13}FO_2$ | 232,259 | Huile | 72,40 | 5,64 | - | 72,26 | 5,82 | - |
| 31 | H | 4-F | $2\text{-}OCOCH_3$ | H | A | $C_{16}H_{15}FO_3$ | 274,296 | Huile | 70,06 | 5,51 | - | 69,89 | 5,40 | - |
| 32 | H | 4-Cl | 2-OH | H | A | $C_{14}H_{13}ClO_2$ | 248,709 | Huile | 67,61 | 5,28 | - | 67,39 | 5,40 | - |
| 33 | H | 4-Cl | $2\text{-}OCOCH_3$ | H | A | $C_{16}H_{15}ClO_3$ | 290,746 | Huile | 66,09 | 5,20 | - | 65,88 | 5,39 | - |
| 34 | H | 4-Cl | 2-OCO | H | A | $C_{19}H_{22}O_3$ | 298,385 | Huile | 76,48 | 7,43 | - | 76,32 | 7,58 | - |
| 35 | H | H | 2-OCO 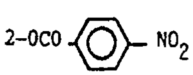 | H | A | $C_{21}H_{17}NO_5$ | 363,373 | 97 | 69,41 | 4,71 | - | 69,29 | 4,72 | - |
| 36 | $5\text{-}CH_3$ | H | 2-OH | H | A | $C_{15}H_{16}O_2$ | 228,294 | Huile | 78,92 | 7,06 | - | 78,80 | 7,01 | - |
| 37 | $5\text{-}CH_3$ | H | $2\text{-}OCOCH_3$ | H | A | $C_{17}H_{18}O_3$ | 270,331 | Huile | 75,53 | 6,71 | - | 75,37 | 6,63 | - |
| 38 | H | H | 2-OCO 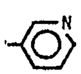 | H | A | $C_{20}H_{17}NO_3$ | 319,363 | 66 | 75,21 | 5,36 | 4,38 | 75,19 | 5,46 | 4,44 |

| EX | $(Z)_p$ | $(Y)_n$ | X | R | METHODE | FORMULE BRUTE | PM | PF°C | ANALYSE CENTESIMALE | | | | | |
|---|---|---|---|---|---|---|---|---|---|---|---|---|---|---|
| | | | | | | | | | CALCULEE % | | | TROUVEE % | | |
| | | | | | | | | | C | H | N | C | H | N |
| 39 | H | H | 2-OCO 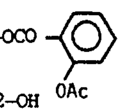 | H | A | $C_{23}H_{20}O_5$ | 376,42 | 72 | 73,39 | 5,35 | - | 73,39 | 5,35 | - |
| 40 | 5-F | 4-F | 2-OH | H | A | $C_{14}H_{12}F_2O_2$ | 250,251 | 68 | 67,19 | 4,83 | - | 67,04 | 4,78 | - |
| 41 | 5-F | 4-F | 2-OAc | H | A | $C_{16}H_{14}F_2O_3$ | 298,288 | | | | | | | |
| 42 | 5-OH | H | 2-OH | H | A | $C_{14}H_{14}O_3$ | 230,266 | 86 | 73,02 | 6,13 | - | 72,87 | 6,27 | - |
| 43 | 5-OAc | H | 2-OAc | H | A | $C_{18}H_{18}O_5$ | 314,34 | Huile | 68,78 | 5,77 | - | 68,61 | 5,81 | - |
| 44 | $4,5(CH_2)_3$ | H | 2-OH | H | A | $C_{17}H_{18}O_2$ | 254,332 | 92 | | | | | | |
| 45 | $4,5(CH_2)_3$ | H | 2-OAc | H | A | $C_{19}H_{20}O_3$ | 296,369 | Huile | | | | | | |
| 46 | 3,4benzo | 4-F | 2-OH | H | A | $C_{18}H_{15}FO_2$ | 282,32 | 80 | 76,58 | 5,35 | - | 76,44 | 5,24 | - |
| 47 | 3,4benzo | 4-F | 2-OAc | H | A | $C_{20}H_{17}FO_3$ | 324,356 | 72 | 74,06 | 5,28 | - | 74,12 | 5,32 | |
| 48 | 5,6benzo | 4-F | 2-OH | H | A | $C_{18}H_{15}FO_2$ | 282,32 | 113 | 76,58 | 5,35 | - | 76,47 | 5,35 | - |
| 49 | 5,6benzo | 4-F | 2-OAc | H | A | $C_{20}H_{17}FO_3$ | 324,356 | 91 | 74,06 | 5,28 | - | 74,12 | 5,33 | - |
| 50 | H | $4-OCH_3$ | 2-OH | H | A | $C_{15}H_{16}O_3$ | 244,293 | 70 | 73,75 | 6,60 | - | 73,69 | 6,54 | - |
| 51 | H | $4-OCH_3$ | $2-OCOCH_3$ | H | A | $C_{17}H_{18}O_4$ | 286,331 | Huile | 71,31 | 6,33 | - | 71,40 | 6,55 | - |
| 52 | 5-t.bu | 4-F | 2-OH | H | B | $C_{18}H_{21}FO_2$ | 288,367 | 86 | 74,97 | 7,34 | - | 75,07 | 7,29 | - |
| 53 | 5-t.bu | 4-F | $2-OCOCH_3$ | H | B | $C_{20}H_{23}FO_3$ | 330,404 | Huile | 72,70 | 7,01 | - | 72,54 | 7,16 | - |
| 54 | 5-Me | 4-F | 2-OH | H | B | $C_{15}H_{15}FO_2$ | 246,286 | Huile | 73,15 | 6,14 | - | 72,97 | 6,19 | - |
| 55 | $5-CH_3$ | 4-F | $2-OCOCH_3$ | H | B | $C_{17}H_{17}FO_3$ | 288,323 | Huile | 70,82 | 5,94 | - | 70,61 | 6,69 | |

| EX | $(Z)_p$ | $(Y)_n$ | X | R | METHODE | FORMULE BRUTE | PM | PF°C | ANALYSE CENTESIMALE | | | | | |
|---|---|---|---|---|---|---|---|---|---|---|---|---|---|---|
| | | | | | | | | | CALCULEE % | | | TROUVEE % | | |
| | | | | | | | | | C | H | N | C | H | N |
| 56 | H | 3,4-benzo | 2-OH | H | B | $C_{18}H_{16}O_2$ | 264,328 | 80 | 81,79 | 6,10 | - | 81,66 | 6,07 | - |
| 57 | H | 3,4-benzo | $2-OCOCH_3$ | H | B | $C_{20}H_{18}O_3$ | 306,364 | 73 | 78,41 | 5,92 | - | 78,33 | 5,81 | - |
| 58 | H | 2,3-benzo | 2-OH | H | B | $C_{18}H_{16}O_2$ | 264,328 | 82 | 81,79 | 6,10 | - | 81,75 | 6,02 | - |
| 59 | H | 2,3-benzo | $2-OCOCH_3$ | H | B | $C_{20}H_{18}O_3$ | 306,364 | ≃ 50 | 78,41 | 5,92 | - | 78,33 | 5,77 | - |
| 60 | H | $3,4-OCH_2O$ | 2-OH | H | B | $C_{15}H_{14}O_4$ | 258,277 | 87 | 69,75 | 5,46 | - | 69,52 | 5,28 | - |
| 61 | H | $3,4-OCH_2O$ | $2-OCOCH_3$ | H | B | $C_{17}H_{16}O_5$ | 300,315 | 57 | 67,99 | 5,37 | - | 68,03 | 5,24 | - |
| 62 | H | $4-CH_3$ | 2-OH | H | B | $C_{15}H_{16}O_2$ | 228,294 | Huile | 78,92 | 7,06 | - | 78,77 | 7,04 | |
| 63 | H | $4-CH_3$ | $2-OCOCH_3$ | H | B | $C_{17}H_{18}O_3$ | 270,331 | Huile | 75,55 | 6,71 | - | 75,38 | 6,90 | - |
| 64 | 4-O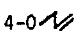 | H | 2-OH | H | A | $C_{17}H_{18}O_3$ | 270,331 | 58 | 75,53 | 6,71 | - | 75,42 | 6,61 | - |
| 65 | 4-O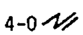 | H | $2-OCOCH_3$ | H | A | $C_{19}H_{20}O_4$ | 312,369 | Huile | 73,05 | 6,45 | - | 72,91 | 6,29 | - |
| 66 | H | 4-F | 2-OCO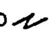$CO_2H$ | H | A | $C_{18}H_{17}FO_5$ | 332,33 | 82 | 65,05 | 5,15 | - | 65,15 | 5,09 | - |
| 67 | $5-OCH_3$ | 4-F | 2-OCO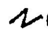$CO_2H$ | H | A | $C_{19}H_{19}FO_6$ | 362,359 | 69 | 62,979 | 5,285 | - | 63,03 | 5,20 | - |
| 68 | H | 4-F | $2-OCH_3$ | H | A | $C_{15}H_{15}FO_2$ | 246,28 | Huile | 73,155 | 6,14 | - | 73,09 | 6,24 | - |
| 69 | $5-OCH_3$ | 4-F | $2-OCH_3$ | H | A | $C_{16}H_{17}FO_3$ | 276,311 | Huile | 69,55 | 6,20 | - | 69,49 | 6,10 | - |
| 70 | H | $2-CCH_3$ | 2-OH | H | R | $C_{15}H_{16}O_3$ | 244,293 | 96 | 73,75 | 6,60 | - | 73,68, | 6,56 | - |
| 71 | H | $2-OCH_3$ | $2-OCOCH_3$ | H | B | $C_{17}H_{18}O_4$ | 286,331 | 70 | 71,31 | 6,33 | - | 71,26 | 6,42 | - |
| 72 | H | $2,6(OCH_3)_2$ | 2-OH | H | B | $C_{16}H_{18}O_4$ | 274,319 | 84 | 70,05 | 6,61 | - | 69,86 | 6,52 | - |
| 73 | H | $2,6(OCH_3)_2$ | $2-OCOCH_3$ | H | B | $C_{18}H_{20}O_5$ | 316,357 | Huile | 68,34 | 6,37 | - | 68,19 | 6,37 | - |

| EX | $(Z)_p$ | $(Y)_n$ | X | R | METHODE | FORMULE BRUTE | PM | PF°C | ANALYSE CENTESIMALE | | | | | |
|---|---|---|---|---|---|---|---|---|---|---|---|---|---|---|
| | | | | | | | | | CALCULEE % | | | TROUVEE % | | |
| | | | | | | | | | C | H | N | C | H | N |
| 74 | H | 2,3-$Cl_2$ | 2-OH | H | B | $C_{14}H_{12}Cl_2O_2$ | 283,161 | 81 | 59,38 | 4,27 | - | 59,43 | 4,34 | - |
| 75 | H | 2,3-$Cl_2$ | 2-$OCOCH_3$ | H | B | $C_{16}H_{14}Cl_2O_3$ | 325,198 | 75 | 59,09 | 4,34 | - | 59,14 | 4,50 | - |
| 76 | H | 3,4,5-$(OCH_3)_3$ | 2-OH | H | B | $C_{17}H_{20}O_5$ | 304,346 | 110 | 67,09 | 6,62 | - | 66,95 | 6,60 | - |
| 77 | H | 3,4,5-$(OCH_3)_3$ | 2-$OCOCH_3$ | H | B | $C_{19}H_{22}O_6$ | 346,383 | ≃ 50 | 65,88 | 6,40 | - | 66,01 | 6,45 | - |
| 78 | 3,4$(CH_3)_2$ | 4-F | 2-OH | H | A | $C_{16}H_{17}FO_2$ , 2 $H_2O$ | 296,343 | 65 | 64,83 | 7,14 | - | 65,12 | 7,0 | - |
| 79 | 3,4$(CH_3)_2$ | 4-F | 2-$OCOCH_3$ | H | A | $C_{18}H_{19}FO_3$ | 302,35 | 61 | 71,50 | 6,33 | - | 71,33 | 6,25 | |
| 80 | 3-iPr | 4-F | 2-OH | H | A | $C_{17}H_{19}FO_2$ | 274,34 | Huile | 74,43 | 6,98 | - | 74,28 | 6,91 | - |
| 81 | 3-iPr | 4-F | 2-$OCOCH_3$ | H | A | $C_{19}H_{21}FO_3$ | 316,377 | 53 | 72,13 | 6,69 | - | 72,03 | 6,72 | |
| 82 | 5-iPr | 4-F | 2-OH | H | A | $C_{17}H_{19}FO_2$ | 274,34 | 61 | 74,43 | 6,98 | - | 74,30 | 6,99 | - |
| 83 | 5-iPr | 4-F | 2-$OCOCH_3$ | H | A | $C_{19}H_{21}FO_3$ | 316,377 | Huile | 72,13 | 6,69 | - | 71,96 | 6,54 | - |
| 84 | H | 4-OH | 2-OH | H | B | $C_{14}H_{14}O_3$ | 230,266 | 90 | 73,02 | 6,12 | - | 72,84 | 6,28 | - |
| 85 | H | 4-$OCOCH_3$ | 2-$OCOCH_3$ | H | B | $C_{18}H_{18}O_5$ | 314,341 | Huile | 68,78 | 5,77 | - | 68,60 | 5,62 | - |
| 86 | 3-nPr | 4-F | 2-OH | H | A | $C_{17}H_{19}FO_2$ | 274,34 | Huile | 74,42 | 6,98 | - | 74,34 | 6,88 | - |
| 87 | 3-nPr | 4-F | 2-$OCOCH_3$ | H | A | $C_{19}H_{21}FO_3$ | 316,377 | Huile | 72,13 | 6,69 | - | 72,06 | 6,74 | - |
| 88 | 5-$OC_2H_5$ | 4-F | 2-OH | H | A | $C_{16}H_{17}FO_3$ | 276,311 | Huile | 69,55 | 6,20 | - | 69,38 | 5,96 | - |

| EX | $(Z)_p$ | $(Y)_n$ | X | R | METHODE | FORMULE BRUTE | PM | PF°C | ANALYSE CENTESIMALE | | | | | |
|---|---|---|---|---|---|---|---|---|---|---|---|---|---|---|
| | | | | | | | | | CALCULEE % | | | TROUVEE % | | |
| | | | | | | | | | C | H | N | C | H | N |
| 89 | 5-$OC_2H_5$ | 4-F | 2-$OCOCH_3$ | H | A | $C_{18}H_{19}FO_4$ | 318,35 | Huile | 67,91 | 6,01 | - | 67,96 | 5,99 | - |
| 90 | H | 4-$CO_2$Et | 2-OH | H | B | $C_{17}H_{18}O_4$ | 286,331 | 72 | 71,31 | 6,34 | - | 71,13 | 6,28 | - |
| 91 | H | 4-$CO_2$Et | 2-$OCOCH_3$ | H | B | $C_{19}H_{20}O_5$ | 328,368 | 60 | 69,50 | 6,14 | - | 69,57 | 6,24 | - |
| 92 | H | 4-$CH_2CO_2$Et | 2-OH | H | B | $C_{18}H_{20}O_4$ | 300,358 | 60 | 71,98 | 6,71 | - | 71,97 | 6,68 | - |
| 93 | H | 4-$CH_2CO_2$Et | 2-$OCOCH_3$ | H | B | $C_{20}H_{22}O_5$ | 342,395 | Huile | 70,15 | 6,47 | - | 70,20 | 6,39 | - |
| 94 | H | 4-$CH_2CO_2H$ | 2-OH | H | B | $C_{16}H_{16}O_4$ | 272,304 | 108 | 70,57 | 5,92 | - | 70,45 | 5,84 | - |
| 97 | H | 4-$CH_2CO_2CH_3$ | 2-$OCH_3$ | H | B | $C_{18}H_{20}O_4$ | 300,358 | Huile | 71,98 | 6,71 | - | 71,81 | 6,67 | - |
| 98 | 5-Ph | 4-F | 2-OH | H | A | $C_{20}H_{17}FO_2$ | 308,35 | Huile | 77,90 | 5,55 | - | 77,69 | 5,42 | - |
| 99 | 5-Ph | 4-F | 2-$OCOCH_3$ | H | A | $C_{22}H_{19}FO_3$ | 350,39 | Huile | 75,41 | 5,46 | - | 75,44 | 5,42 | - |
| 100 | 5-OPh | 4-F | 2-OH | H | A | $C_{20}H_{17}FO_3$ | 324,35 | Huile | 74,06 | 5,28 | - | 72,77 | 5,21 | - |
| 101 | 5-OPh | 4-F | 2-$OCOCH_3$ | H | A | $C_{22}H_{19}FO_4$ | 366,39 | Huile | 72,12 | 5,22 | - | 72,18 | 5,20 | - |
| 102 | 5-OH | 4-F | 2-OH | H | A | $C_{14}H_{13}FO_3$ | 248,259 | 88 | 67,73 | 5,27 | - | 67,56 | 5,42 | - |
| 103 | 5-$OCOCH_3$ | 4-F | 2-$OCOCH_3$ | H | A | $C_{18}H_{17}FO_5$ | 332,333 | 76 | 65,05 | 5,15 | - | 65,20 | 5,31 | - |
| 104 | 5-$OCO_2$Et | H | 2-OH | H | A | $C_{17}H_{18}O_5$ | 302,23 | 55 | 67,56 | 6,00 | - | 67,52 | 5,95 | - |
| 105 | 5-$OCO_2$Et | H | 2-$OCOCH_3$ | H | A | $C_{19}H_{20}O_6$ | 344,367 | Huile | 66,27 | 5,85 | - | 66,17 | 5,76 | - |
| 106 | H | 4-F | 2-$OSO_3$Na | H | A | $C_{14}H_{12}FO_5SNa$ | 334,30<br>352,31 | 78-81 | 47,73 | 4,00 | - | 47,76 | 3,85 | - |

| EX | $(Z)_p$ | $(Y)_n$ | X | R | METHODE | FORMULE BRUTE | PM | PF°C | ANALYSE CENTESIMALE CALCULEE % C | CALCULEE % H | CALCULEE % N | TROUVEE % C | TROUVEE % H | TROUVEE % N |
|---|---|---|---|---|---|---|---|---|---|---|---|---|---|---|
| 107 | 4,5-O-$(CH_2)_2O$ | 4-F | 2-OH | H | A | $C_{16}H_{15}FO_4$ | 290,296 | 89 | 66,2 | 5,21 | - | 66,08 | 5,37 | - |
| 108 | 4,5-O-$(CH_2)_2O$ | 4-F | 2-$OCOCH_3$ | H | A | $C_{18}H_{17}FO_5$ | 332,333 | 59 | 65,05 | 5,15 | - | 65,00 | 5,06 | - |
| 109 | 4,5-O-$(CH_2)_2O$ | 4-F | 2-$OCH_3$ | H | A | $C_{17}H_{17}FO_4$ | 304,323 | 61 | 67,10 | 5,63 | - | 66,98 | 5,70 | - |
| 110 | 5-$OCH_3$ | 4-iPr | 2-OH | H | A | $C_{18}H_{22}O_3$ | 286,374 | Huile | 75,49 | 7,74 | - | 75,33 | 7,65 | - |
| 111 | 5-$OCH_3$ | 4-iPr | 2-$OCOCH_3$ | H | A | $C_{20}H_{24}O_4$ | 328,41 | Huile | 73,14 | 7,36 | - | 73,24 | 7,29 | - |
| 112 | 5-$OCH_3$ | 4-iPr | 2-$OCH_3$ | H | A | $C_{19}H_{24}O_3$ | 300,40 | Huile | 75,97 | 8,05 | - | 75,88 | 7,91 | - |
| 113 | 5-$CH_3$ | H | 2-$OSO_3Na$ | H | A | $C_{15}H_{15}O_5SNa$ | 330,33 | pâteux à partir 50 | 54,54 | 4,57 | - | 54,27 | 4,70 | - |
| 114 | 4-$CH_3$ | 4-F | 2-OH | H | A | $C_{15}H_{15}FO_2$ | 246,28 | 66 | 73,15 | 6,14 | - | 73,02 | 6,12 | - |
| 115 | 4-$CH_3$ | 4-F | 2-$OCOCH_3$ | H | A | $C_{17}H_{17}FO_3$ | 288,32 | Huile | 70,82 | 5,94 | - | 70,78 | 5,84 | - |
| 116 | 4-$CH_3$ | 4-F | 2-$OCH_3$ | H | A | $C_{16}H_{17}FO_2$ | 260,31 | Huile | 73,82 | 6,58 | - | 73,81 | 6,51 | - |
| 117 | 4-$OCH_3$ | 4-F | 2-OH | H | A | $C_{15}H_{15}FO_3$ | 262,285 | 62 | 68,69 | 5,76 | - | 68,63 | 5,82 | - |
| 118 | 4-$OCH_3$ | 4-F | 2-$OCOCH_3$ | H | A | $C_{17}H_{17}FO_4$ | 304,323 | 54 | 67,09 | 5,63 | - | 66,91 | 5,57 | - |
| 119 | 4-$OCH_3$ | 4-F | 2-$OCH_3$ | H | A | $C_{16}H_{17}FO_3$ | 276,311 | 70 | 69,55 | 6,20 | - | 69,41 | 6,14 | - |
| 120 | 3-OH | 4-F | 2-$OCH_3$ | H | C | $C_{15}H_{15}FO_3$ | 262,285 | Huile | 68,69 | 5,76 | - | 68,63 | 5,75 | - |
| 121 | 3-$OCOCH_3$ | 4-F | 2-$OCH_3$ | H | C | $C_{17}H_{17}FO_4$ | 304,323 | 63 | 67,096 | 5,63 | - | 67,16 | 5,70 | - |

| EX | (Z)p | (Y)n | X | R | METHODE | FORMULE BRUTE | PM | PF°C | ANALYSE CENTESIMALE | | | | | |
|---|---|---|---|---|---|---|---|---|---|---|---|---|---|---|
| | | | | | | | | | CALCULEE % | | | TROUVEE % | | |
| | | | | | | | | | C | H | N | C | H | N |
| 122 | 4-$CH_3$ | H | 2-OH | H | A | $C_{15}H_{16}O_2$ | 228,294 | < 50 | 78,92 | 7,06 | - | 78,86 | 7,06 | - |
| 123 | 4-$CH_3$ | H | 2-$OCOCH_3$ | H | A | $C_{17}H_{18}O_3$ | 270,33 | Huile | 75,53 | 6,71 | - | 75,31 | 6,55 | - |
| 124 | 4-$CH_3$ | H | 2-$OCH_3$ | H | A | $C_{16}H_{18}O_2$ | 242,32 | Huile | 79,30 | 7,48 | - | 79,28 | 7,40 | - |
| 125 | H | 4-F | 2-OCO 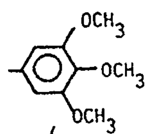 | H | A | $C_{24}H_{23}FO_6$ | 426,44 | 96 | 67,60 | 5,44 | - | 67,42 | 5,41 | - |
| 126 | H | 4-F | 2-O 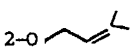 | H | A | $C_{19}H_{21}FO_2$ | 300,378 | Huile | 75,97 | 7,04 | - | 75,78 | 6,96 | - |
| 127 | H | 4-F | 2-OCO 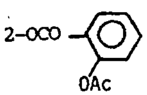 | H | A | $C_{23}H_{19}FO_5$ | 394,40 | 91 | 70,04 | 4,85 | - | 69,89 | 4,97 | - |
| 128 | H | 4-F | 2-$OSO_2NH_2$ | H | A | $C_{14}H_{14}FNO_4S$ | 311,33 | Huile | 54,01 | 4,53 | 4,50 | 53,82 | 4,69 | 4,47 |
| 129 | H | 4-F | 2-OCO 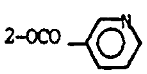 | H | A | $C_{20}H_{17}ClFNO_3$ | 373,82 | 155 | 64,26 | 4,58 | 3,74 | 64,47 | 4,75 | 3,70 |
| 130 | H | 4-F | 2-OCO 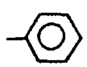 | H | A | $C_{21}H_{17}FO_3$ | 336,367 | Huile | 74,98 | 5,09 | - | 74,80 | 4,93 | - |
| 131 | H | 4-F<br>2-OCO 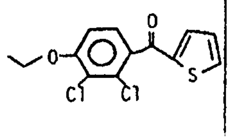 | | H | A | $C_{27}H_{19}Cl_2FO_5S$ | 545,419 | 128 | 59,46 | 3,51 | - | 59,41 | 3,62 | - |

| EX | (Z)p | (Y)n | X | R | METHODE | FORMULE BRUTE | PM | PF°C | ANALYSE CENTESIMALE | | | | | |
|---|---|---|---|---|---|---|---|---|---|---|---|---|---|---|
| | | | | | | | | | CALCULEE % | | | TROUVEE % | | |
| | | | | | | | | | C | H | N | C | H | N |
| 132 | H | 4-F | 2-OCO 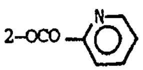 | H | A | $C_{20}H_{17}ClFNO_3$ | 373,82 | 125 | 64,26 | 4,58 | 3,74 | 64,29 | 4,53 | 3,72 |
| 133 | H | 4-F | 2-OCO 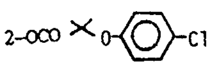 | H | A | $C_{24}H_{22}ClFO_4$ | 428,89 | Huile | 67,21 | 5,17 | - | 67,12 | 5,26 | - |
| 134 | H | 4-F | 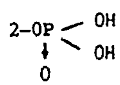 | H | A | $C_{14}H_{14}FO_5P$ | 312,23 | 82 | 53,86 | 4,52 | - | 53,71 | 4,62 | - |
| 135 | 5-$CH_3$ | H | 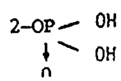 | H | A | $C_{15}H_{17}O_5P$ | 308,269 | 104 | 58,44 | 5,55 | - | 58,32 | 5,67 | - |
| 136 | H | 4-F | 2-OCO 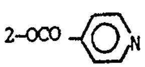 | H | A | $C_{20}H_{17}ClFNO_3$ | 373,82 | 152 | 64,26 | 4,58 | 3,74 | 64,12 | 4,64 | 3,66 |
| 137 | H | 4-F | 2-OCO 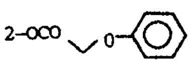 | H | A | $C_{22}H_{19}FO_4$ | 366,39 | 52 | 72,12 | 5,22 | - | 71,98 | 5,33 | - |
| 138 | H | 4-F | 2-OCO 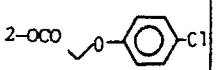 | H | A | $C_{22}H_{18}ClFO_4$ | 400,84 | 70 | 65,92 | 4,52 | - | 65,83 | 4,64 | - |
| 139 | H | 4-F | 2-OCO 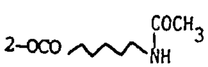 | H | A | $C_{22}H_{26}FNO_4$ | 387,45 | 78 | 68,20 | 6,76 | 3,61 | 68,04 | 6,79 | 3,62 |
| 140 | 3,6($OCH_3$)$_2$ | H | 2-OH | H | A | $C_{16}H_{18}O_4$ | 274,31 | Huile | 70,05 | 6,61 | - | 70,12 | 6,64 | - |

| EX | (Z)p | (Y)n | X | R | METHODE | FORMULE BRUTE | PM | PF°C | ANALYSE CENTESIMALE CALCULEE % C | H | N | TROUVEE % C | H | N |
|---|---|---|---|---|---|---|---|---|---|---|---|---|---|---|
| 141 | 3,6(OCH$_3$)$_2$ | H | -OCH$_3$ | H | A | $C_{17}H_{20}O_4$ | 288,34 | Huile | 70,81 | 6,99 | - | 70,74 | 6,88 | - |
| 142 | H | 4-F | 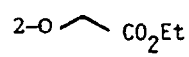 | H | A | $C_{18}H_{19}FO_4$ | 318,35 | Huile | 67,91 | 6,01 | - | 68,02 | 6,12 | - |
| 143 | H | 4-F | 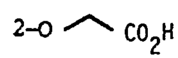 | H | A | $C_{16}H_{15}FO_4$ | 290,29 | 112 | 66,20 | 5,20 | - | 66,3 | 5,31 | - |
| 144 | 5-CH$_2$CO$_2$CH$_3$ | 4-F | 2-OH | H | B | $C_{17}H_{17}FO_4$ | 304,323 | 70 | 67,10 | 5,63 | - | 66,32 | 5,57 | - |
| 145 | 5-CH$_2$CO$_2$H | 4-F | 2-OH | H | B | $C_{16}H_5FO_4$ | 290,296 | 127 | 66,20 | 5,21 | - | 66,03 | 5,19 | - |
| 146 | 3,4-Cl$_2$ | 4-F | 2-OH | H | B | $C_{14}H_{11}Cl_2FO_2$ | 301,15 | 68 | 55,83 | 3,68 | - | 55,77 | 3,69 | - |
| 147 | 3,4-Cl$_2$ | 4-F | 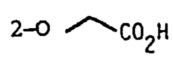 | H | B | $C_{16}H_{13}Cl_2FO_4$ | 359,19 | 105 | 53,50 | 3,64 | - | 53,41 | 3,68 | - |
| 148 | 3,6(OCH$_3$)$_2$ | 4-F | 2-OH | H | A | $C_{16}H_{17}FO_4$ | 292,31 | 68 | 65,74 | 5,86 | - | 65,53 | 5,88 | - |
| 149 | 5-CH$_3$ | 4-F | 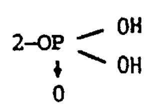 | H | A | $C_{15}H_{18}FPO_7$ | 344,26 | 63 | 52,28 | 5,22 | - | 52,03 | 4,95 | - |
| 150 | 5-CH$_3$ | 4-OH | 2-OH | H | B | $C_{15}H_{16}O_3$ | 244,29 | 86 | 73,75 | 6,60 | - | 73,58 | 6,66 | - |
| 151 | 5-F | H | 2-OH | H | B | $C_{14}H_{13}FO_3$ | 232,259 | < 50 | 72,40 | 5,64 | - | 72,29 | 5,76 | - |
| 152 | 5-Cl | 4-F | 2-OH | H | B | $C_{14}H_{12}ClFO_2$ | 266,701 | Huile | 63,05 | 4,53 | - | 62,88 | 4,66 | - |
| 153 | 5-Cl | H | 2-OH | H | B | $C_{14}H_{13}ClO_2$ | 248,709 | Huile | 67,61 | 5,27 | - | 67,45 | 5,40 | - |

| EX | $(Z)_p$ | $(Y)_n$ | X | R | METHODE | FORMULE BRUTE | PM | PF°C | ANALYSE CENTESIMALE | | | | | |
|---|---|---|---|---|---|---|---|---|---|---|---|---|---|---|
| | | | | | | | | | CALCULEE % | | | TROUVEE % | | |
| | | | | | | | | | C | H | N | C | H | N |
| 160 | H | 3,4-$F_2$ | 2-OH | H | B | $C_{14}H_{12}F_2O_2$ | 250,251 | < 50 | 67,19 | 4,83 | - | 67,18 | 5,01 | - |
| 161 | H | 2,5-$F_2$ | 2-OH | H | B | $C_{14}H_{12}F_2O_2$ | 250,251 | 72 | 67,19 | 4,83 | - | 66,99 | 5,02 | - |
| 162 | H | 2,4-$F_2$ | 2-OH | H | B | $C_{14}H_{12}F_2O_2$ | 250,251 | < 50 | 67,19 | 4,83 | - | 67,22 | 4,99 | - |
| 163 | 5-$CONH_2$ | 4-F | 2-OH | H | B | $C_{15}H_{14}FNO_3$ | 275,284 | 168 | 65,44 | 5,126 | 5,088 | 65,32 | 5,26 | 5,01 |
| 164 | H | 2-OH | 2-OH | H | B | $C_{14}H_{14}O_3$ | 230,266 | 102 | 73,026 | 6,128 | - | 72,8 | 6,25 | - |
| 165 | H | 3-$OCH_3$ | 2-OH | H | B | $C_{15}H_{16}O_3$ | 244,293 | Huile | 73,75 | 6,6 | - | 73,87 | 6,74 | - |
| 166 | H | 3-OH | 2-OH | H | B | $C_{14}H_{14}O_3$ | 230,266 | 86 | 73,02 | 6,12 | - | 72,83 | 6,32 | - |
| 167 | 3-$OCH_3$ | 4-F | 4-$OCOCH_3$ | H | C | $C_{17}H_{17}FO_4$ | 304,323 | 68 | 67,096 | 5,63 | - | 67,01 | 5,68 | - |
| 168 | H | H | 3-$OCH_3$ | H | D | $C_{15}H_{16}O_2$ | 228,294 | Huile | 78,92 | 7,06 | - | 78,69 | 6,85 | - |
| 169 | H | 4-F | 3-$OCH_3$ | H | D | $C_{15}H_{15}FO_2$ | 246,294 | Huile | 73,15 | 6,14 | - | 72,98 | 6,01 | - |
| 170 | H | H | 2-OH | $CH_3$ | B | $C_{15}H_{16}O_2$ | 228,294 | Huile | 78,92 | 7,06 | - | 78,76 | 7,21 | - |
| 171 | H | H | 2-$OCOCH_3$ | $CH_3$ | B | $C_{17}H_{18}O_3$ | 270,331 | Huile | 75,53 | 6,71 | - | 75,37 | 6,81 | - |

| EX | $(Z)_p$ | $(Y)_n$ | X | R | METHODE | FORMULE BRUTE | PM | PF°C | ANALYSE CENTESIMALE CALCULEE % C | H | N | TROUVEE % C | H | N |
|---|---|---|---|---|---|---|---|---|---|---|---|---|---|---|
| 172 | H | 4-F | 2-OH | $CH_3$ | B | $C_{15}H_{15}FO_2$ | 246,286 | Huile | 73,15 | 6,14 | - | 73,01 | 6,03 | - |
| 173 | H | 4-F | $2-OCOCH_3$ | $CH_3$ | B | $C_{17}H_{17}FO_3$ | 288,323 | Huile | 70,82 | 5,94 | - | 70,88 | 6,07 | - |
| 174 | H | 4-F | 3-OH | H | D | $C_{14}H_{13}FO_2$ | 232,26 | 64 | 72,40 | 5,64 | - | 72,38 | 5,55 | - |
| 175 | H | 4-F | $3-OCOCH_3$ | H | D | $C_{16}H_{15}FO_3$ | 274,296 | Huile | 70,06 | 5,51 | - | 70,15 | 5,57 | - |
| 176 | H | 3-F | $3-OCH_3$ | H | D | $C_{15}H_{15}FO_2$ | 246,284 | Huile | 73,15 | 6,14 | - | 73,07 | 5,99 | - |
| 177 | H | $2-COCH_3$ | $3-OCH_3$ | H | D | $C_{17}H_{18}O_3$ | 270,331 | 78 | 75,53 | 6,71 | - | 75,53 | 6,92 | |
| 178 | H | $2-CHOHCH_3$ | $3-OCH_3$ | H | D | $C_{17}H_{20}O_3$ | 272,347 | 39 | 74,97 | 7,40 | - | 74,77 | 7,38 | - |
| 180 | H | 4-F | 3-OH | $CH_3$ | B | $C_{15}H_{15}O_2F$ | 246,286 | Huile | 73,15 | 6,14 | - | 73,01 | 6,26 | - |
| 181 | H | 4-F | $3-OCOCH_3$ | $CH_3$ | B | $C_{17}H_{17}FO_3$ | 288,323 | Huile | 70,82 | 5,94 | - | 70,89 | 6,03 | - |
| 182 | H | 4-F | $3-OCH_3$ | $CH_3$ | B | $C_{16}H_{17}O_2F$ | 260,312 | Huile | 73,83 | 6,58 | - | 73,66 | 6,45 | - |
| 183 | $3-OCH_3$ | 4-F | 4-OH | H | C | $C_{15}H_{15}FO_3$ | 262,285 | Huile | 68,69 | 5,76 | - | 68,56 | 5,80 | - |

d'urine sont déterminés toutes les heures. Le dosage de sodium et du potassium est effectué par spectrophotométrie.

Le Tableau I rassemble les principaux résultats obtenus.

T A B L E A U  I

| PRODUIT | ACTIVITE DIURETIQUE | |
|---|---|---|
| | Pouvoir diurétique en % R = RAT S = SOURIS (x) = Dose minimale active mg/kg PO | Rapport Na/K en % R = RAT S = SOURIS (x) = Dose minimale active mg/kg PO |
| ex 25 | R (12) 190 %<br>S (25) 10 % | R (12) 46 %<br>S (25) 50 % |
| ex 26 | R (50) 250 %<br>S (100) 30 % | R (50) 40 %<br>S (100) 210 % |
| ex 30 | R (12) 170 %<br>S (25) 40 % | R (12) 40 %<br>S (25) 210 % |
| ex 36 | R (100) 140 %<br>S (100) 40 % | R (100) 65 %<br>S (100) 180 % |
| ex 42 | R (12) 190 %<br>S (100) Inactif | R (12) 60 %<br>Inactif |
| ex 169 | R (12) 70 %<br>S (100) Inactif | R (12) 85 %<br>S (100) Inactif |
| ex 46 | R : Inactif<br>S : Inactif | R : Inactif<br>S : Inactif |
| ex 48 | R (25) 80 %<br>S (100) 20 % | R (25) 80 %<br>S (100) 130 % |
| ex 172 | R (25) 80 %<br>S (100) 68 % | R (25) 160 %<br>S (100) 208 % |
| ex 56 | R : Inactif<br>S : Inactif | R : Inactif<br>S : Inactif |
| ex 107 | R (25) 140 %<br>S (100) 80 % | R (25) 60 %<br>S (100) 220 % |
| ex 134 | R (6) 100 %<br>S (25) 40 % | R (6) 50 %<br>S (25) 140 % |
| ex 139 | R (25) 32 %<br>S (25) 40 % | R (25) 80 %<br>S (25) 80 % |
| ex 163 | R (6) 260 %<br>S (25) 34 % | R (6) 53 %<br>S (25) 134 % |
| FUROSEMIDE | R (12) 30 %<br>S (25) 120 % | R (12) 10 %<br>S (25) 320 % |

**B - Action anti-hypertensive sur le rat non anesthésié**

L'étude a été effectuée sur des lots de rats mâles SHR (souche OKAMOTO) âgés de 16 semaines. La pression artérielle systolique est déterminée par la méthode sphygmomanométrique (électro sphygmographe NARCO biosystems type PE 300). Les mesures de tension sont réalisées 24 heures après administration d'un composé de formule générale I, la pression artérielle systolique est calculée à partir de la moyenne de 6 à 8 mesures.

Les résultats obtenus sont rassemblés dans le Tableau II.

TABLEAU II

| PRODUIT | ACTIVITE ANTI-HYPERTENSIVE ETUDE CHEZ LE RAT SHR | |
|---|---|---|
| | A = Aigu<br>C = chronique<br>(x) = Dose étudiée en MG/kg PO | Variation de l'effet anti-hyper-tenseur 24 heures après produit |
| ex 25 | Non testé | Non testé |
| ex 26 | Non testé | Non testé |
| ex 30 | A = (7,5)<br>A = (30)<br>C = (15) | - 11 mmHg<br>- 30 mmHg<br>- 16 mmHg |
| ex 36 | A = (100)<br>C = (100) | Non actif<br>- 20 mmHg |
| ex 42 | Non testé | Non testé |
| ex 169 | Non testé | Non testé |
| ex 46 | Non testé | Non testé |
| ex 48 | A = (60) | - 24 mmHg |
| ex 172 | A = (60) | - 16 mmHg |
| ex 56 | Non testé | Non testé |
| ex 107 | Non testé | Non testé |
| ex 134 | A = (15)<br>A = (60)<br>C = (30) | - 11 mmHg<br>- 37 mmHg<br>- 26 mmHg |
| ex 139 | A = (60) | - 12 mmHg |
| ex 163 | A = (10)<br>A = (60) | - 19 mmHg<br>- 33,5 mmHf |
| FUROSEMIDE | FUROSEMIDE<br>(60)<br>HYDROCHLOROTHIAZIDE<br>(60) | FUROSEMIDE<br>non actif<br>HYDROCHLOROTHIAZIDE<br>non actif |

La cinétique de l'action anti-hypertensive est dissociée de celle de l'effet diurétique.

## C - Action anti-agrégante plaquettaire

Les études ont été réalisées sur des plasmas riches en plaquettes provenant de sang de rat, l'agrégation étant induite par addition d'acide Adénosine diphosphorique. Les courbes néphélométriques

d'agrégation sont obtenues en utilisant un agrégomètre de type BORN, le plasma étant placé en incubation à 37° avec agitation continue de 1100 t/mn (cf. BORN G.V.R et CROSS J. - J. Physiol. 168 (1962) 178.

Le tableau III rassemble les résultats obtenus avec les composés de formule générale I testés.

TABLEAU III

**ACTIVITE ANTIAGREGANTE PLAQUETTAIRE "IN VITRO"**
(Agent agrégant ADP)

| PRODUIT | V à 50 | M à 50 |
|---|---|---|
| TICLOPIDINE | 0,75 mM | 0,85 mM |
| Ex 56 | 0,45 | 0,30 |
| Ex 30 | $0{,}5^{-1}$ | 0,8 |
| Ex 174 | 0,55 | 0,40 |
| Ex 90 | 0,60 | 0,50 |
| Ex 82 | 0,65 | 0,55 |
| Ex 52 | 0,70 | 0,75 |
| Ex 64 | 0,70 | 0,50 |
| Ex 78 | 0,70 | 0,70 |
| Ex 58 | 0,75 | 0,60 |
| Ex 129 | 0,8 | 0,6 |
| Ex 84 | 0,8 | 0,75 |
| Ex 102 | 1,0 | 0,7 |
| Ex 42 | 1,0 | 1,2 |
| Ex 172 | 1,0 | 0,95 |
| Ex 86 | 1,25 | 1,55 |
| Ex 134 | 1,5 | 1,3 |
| Ex 81 | 1,6 | 1 |
| Ex 12 | 1,95 | 1,60 |
| Ex 100 | | 1,1 |
| Ex 169 | > 2 | inactif |
| Ex 54 | > 2 | inactif |
| Ex 36 | > 2 | inactif |

Les résultats in vitro ont été confirmés par des résultats obtenus ex vivo sur des rats recevant pendant 3 jours le composé de formule générale I par voie orale.

On constate une inhibition de l'agrégation plaquettaire qui se traduit par un doublement de la dose d'ADP par rapport au lot témoin pour obtenir une agrégation à 50 %.

La Ticlopidine prise comme substance de référence, à dose identique, donne des résultats similaires.

**D - Action sur les systèmes de transport du sodium et du potassium des érythrocytes humains**

Cet effet a été étudié selon la méthodologie décrite par G. GARAY et coll. dans Naunyn-Schmidberg's Arch. Pharmakol. 334 (1986) 202-209 et dans Bioch. Pharm. 33 (1984) 2013-2020.

Les résultats suivants ont été obtenus et sont rassemblés dans le tableau IV. Ils apparaissent supérieurs à ceux obtenus avec le furosémide et avec le xipamide.

## TABLEAU IV

**EFFET PHARMACOLOGIQUE SUR LES SYSTEMES DE TRANSPORT du $NA^+$ et du $K^+$ ET DES ERYTHROCYTES HUMAINS**

| PRODUITS | EFFET SUR LE COTRANSPORT Na-K IC 50 µM | EFFET SUR L'ECHANGEUR D'ANIONS IC 50 µM |
|---|---|---|
| ex 25 | > 600 | 120 |
| ex 26 | 70 | 40 |
| ex 30 | 170 | 4 |
| ex 36 | > 600 | 0,8 |
| ex 42 | 40 | 12 |
| ex 169 | Non actif | Non actif |
| ex 46 | 95 | 200 |
| ex 41 | ≃ 400 | 200 |
| ex 172 | ≃ 400 | 75 |
| ex 56 | 95 | 200 |
| ex 107 | Non testé | Non testé |
| ex 134 | Non actif | Non actif |
| ex 139 | Non testé | Non testé |
| ex 163 | Non testé | Non testé |
| BUMETANIDE | 0,66 | 300 |
| FUROSEMIDE | 37 | 200 |
| XIPAMIDE | - | 25 |

E - Action anti-lipoxygénase

L'action anti-lipoxygénase a été étudiée sur la lipoxygénase de soja selon la méthode décrite par W.L SMITH et W.E LANDS (J. Biol. Chem. 247 (1972) 1038-1047)

Les résultats suivants ont été obtenus et sont rassemblés dans le Tableau V.

TABLEAU V

| PRODUIT | ACTIVITE ANTI-LIPOXYGENASE IC 50 µM |
|---|---|
| ex 25 | 6 |
| ex 26 | 6 |
| ex 30 | ≃ 100 |
| ex 36 | Inactif |
| ex 42 | Non testé |
| ex 169 | Inactif |
| ex 46 | 0,7 |
| ex 48 | 30 |
| ex 172 | Non testé |
| ex 56 | 25 |
| ex 107 | 7 |
| ex 134 | > 166 |
| ex 139 | Non testé |
| ex 163 | Non testé |
| NDGA | 0,5 |

## Revendications

1°- De nouveaux phenoxyethyl benzènes de formule générale I

$$Ar - \underset{\displaystyle |}{\overset{R}{}}\!\!CH - CH(R_1) - O - Ar' \qquad (I)$$

dans laquelle Ar est un radical aromatique mono- ou bicyclique hydroxylé choisi parmi les dérivés benzéniques de formule

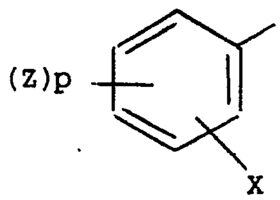

dans laquelle X représente un hydroxyle libre, estérifié par un acide carboxylique, ou un acide sulfonique, par l'acide phosphorique, ou par l'acide sulfurique ou ethérifié par un alcoyle inférieur ou un alcényle inférieur

Z est un substituant choisi dans le groupe constitué par l'hydrogène, les halogènes, un hydroxy, un alcoxy inférieur, un acyloxy, un phényl alcoyle inférieur, un alcoyle inférieur, un alcoyle inférieur substitué par un (alcoyl inférieur)oxycarbonyle ou par un hydroxy carbonyle, une chaine $CO(CH_2)_M\ CH_3$ dans laquelle m représente un nombre entier allant de 1 à 4, une chaine $CHOH\text{-}(CH_2)_{m'}\ CH_3$ dans laquelle m′ représente un nombre entier variant de 1 à 4, un phényle, un phénoxy et un groupe carboxamido éventuellement substitué par un ou deux radicaux alcoyle inférieur et parmi les dérivés naphtaléniques choisis dans le groupe constitué parmi les composés de formule

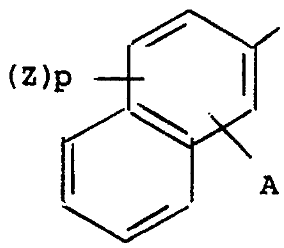

dans laquelle A est un hydroxyle libre, ethérifié ou estérifié

Z est défini comme précédemment

et p est un nombre entier variant de 1 à 3

et ceux de formule

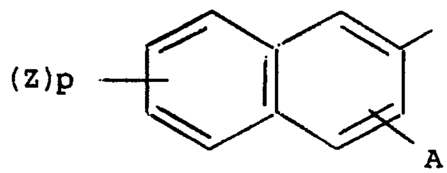

dans laquelle A, Z et p sont définis comme précédemment

Ar′ est un radical aromatique mono- ou bicyclique choisi dans le groupe constitué parmi

- les dérivés benzéniques de formule

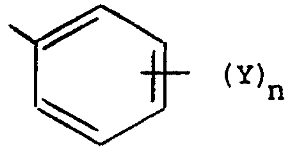

dans laquelle Y représente de l'hydrogène, un halogène, un alcoylène dioxy, un hydroxy, un alcoxy inférieur, un acyloxy dérivé d'u acide organique carboxylique, un alcoyle inférieur, un alcoyle inférieur substitué par un radical (alcoxy inférieur) carbonyle, une chaine oxo alcoyle de formule $CO(CH_2)_m\text{-}CH_3$ dans laquelle m représente un nombre entier allant de 1 à 4 et une chaine hydroxy alcoyle de formule $CHOH\text{-}(CH_2)_{m'}\text{-}CH_3$ dans laquelle m′ représente un nombre entier allant de 1 à 4.

- les dérivés naphtaléniques choisis dans le groupe constitué par les dérivés de formule

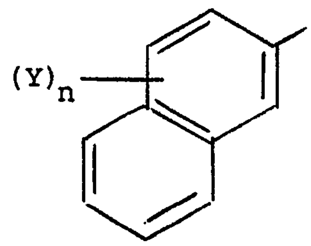

dans laquelle Y est défini comme précédemment
et n est un nombre entier variant de 1 à 3
- et les dérivés de formule

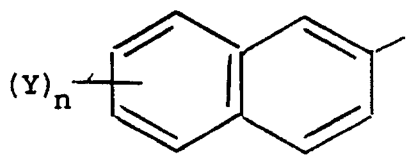

dans laquelle Y est défini comme précedemment
et n est un nombre entier variant de 1 à 3
R est un hydrogène, un hydroxyle, l'oxygène d'une fonction carbonylée ou un radical alcoyle inférieur
$R_1$ est un hydrogène ou un radical alcoyle inférieur

2°- Les formes optiquement-actives des compos de formule générale I lorsque R ou $R_1$ sont différents de l'hydrogène et que R ou $R_1$ représentent un hydroxyle ou un radical alcoyle inférieur.

3°- Les sels avec un métal alcalin ou alcalino terreux ou avec une base organique des composés de formule I lorsque X représente un hydroxyle estérifié par l'acide phosphorique ou sulfurique.

4°- Les composés de formule générale I selon l'une des revendications 1 à 3 ayant la formule générale $I_A$

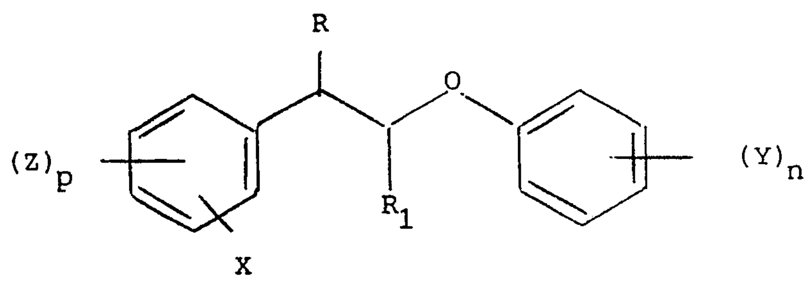

$(I_A)$

dans laquelle X est un hydroxyle libre, éthérifié par un alcoyle inférieur ou un alcenyle inférieur ou esérifié par un acide organique carboxylique ou sulfonique, par l'acide sulfurique ou par l'acide phosphorique
R, $R_1$, Y, Z, n et p gardent les significations fournies précédemment.

5°- Un composé selon l'une des revendications 1 à 4° à savoir le 1-(2-hydroxy 5-méthoxy phényl) 2-phénoxy éthane

6°- Un composé selon la revendication 5° à savoir les phénates métalliques ou organiques du 1-(2-hydroxy 5-méthoxyphényl) 2-phénoxy éthane.

7°- Un composé selon l'une des revendications 1 à 4° à savoir le 1-(2-hydroxy 5-carboxamido phényl) 2-(4-fluorophénoxy) éthane.

8°- Les composés de formule générale I selon l'une des revendications 1 à 3° répondant à la formule générale $I_B$

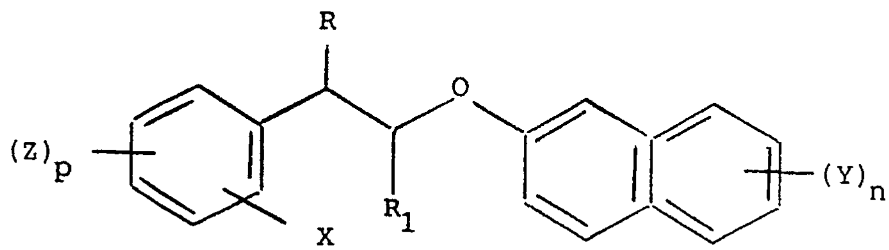

$(I_B)$

dans laquelle la définition des substituants Y, Z, R, $R_1$, n et p demeure inchangée.

9°- Les composés selon l'une des revendications 1 à 3° répondant à la formule $I_C$

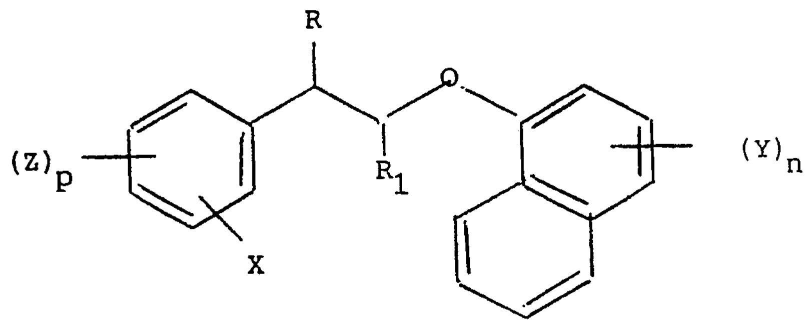

$(I_C)$

dans laquelle la définition des substituants Z, Y, R, $R_1$, X, n et p demeure inchangée.

10°- Les composés selon l'une des revendications 1 à 3° répondant à la formule $I_D$

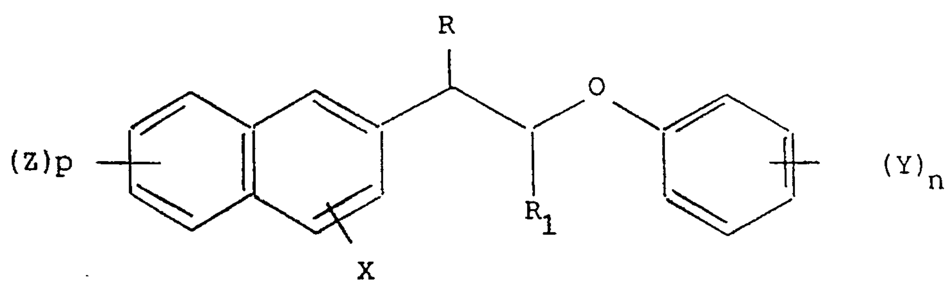

$(I_D)$

dans laquelle Z, Y, X, R, $R_1$, n et p sont définis comme précédemment.

11° Les composés selon l'une des revendications 1 à 3° répondant à la formule $I_E$

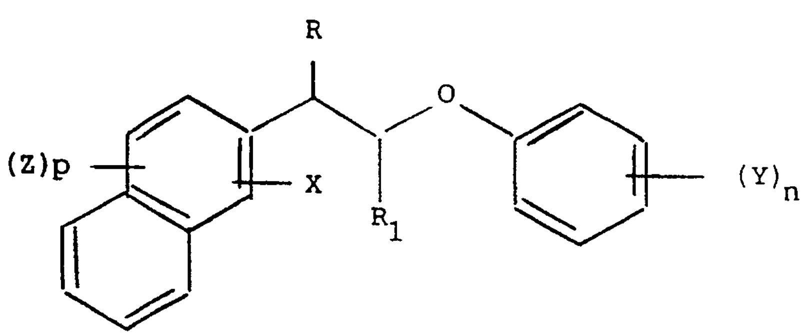

$(I_E)$

dans laquelle R, $R_1$, X, Y, Z, n et p ont les significations fournies précédemment.

12°- Un procédé d'obtention des composés de formule générale I selon l'une des revendications 1 à 11°

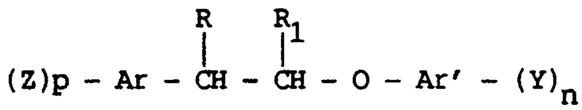

(I)

dans laquelle Ar, R, $R_1$ et Ar′ ont les définitions fournies précédemment caractérisé en ce que l'on condense un phénol de formule générale II

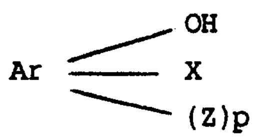

(II)

dans laquelle Ar, X, Z et p sont définis comme précédemment avec un aryl acétonitrile de formule générale III

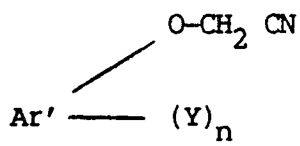

(III)

dans laquelle Y, Ar′ et n sont définis comme précédemment en présence d'un catalyseur acide pour former une diaryl éthanone de formule générale IV

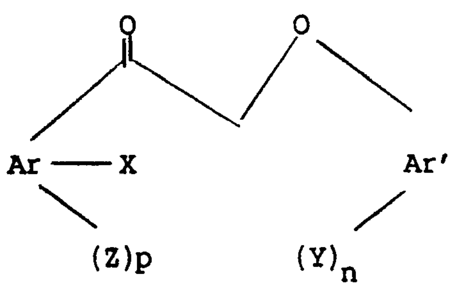

(IV)

dans laquelle Z, Y, Ar, Ar′, n et p sont définis comme précedemment et X est un hydroxyle libre que l'on réduit ensuite par action d'un hydrure mixte de métal alcalin en présence d'un catalyseur métallique ou par réaction avec un chloroformiate d'alcoyle inférieur puis réduction du produit formé intermédiairement, par un borohydrure de métal alcalin pour obtenir un composé de formule générale I

13°- Un procédé d'obtention des composés de formule générale I selon l'une des revendications 1 à 11° qui consiste en ce que l'on condense un phénol de formule générale $II_A$

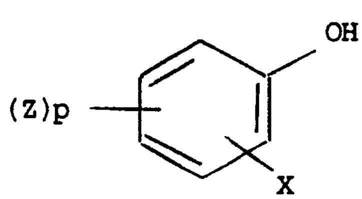

($II_A$)

dans laquelle X, Z et p sont éfinis comme précedemment avec un phénoxy acéto-nitrile de formule générale $III_A$

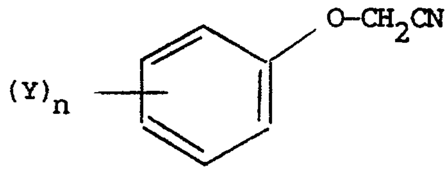

(III$_A$)

dans laquelle Y et n ont les définitions antérieures
en présence d'un catalyseur acide, pour former l'éthanone de formule générale IV$_A$

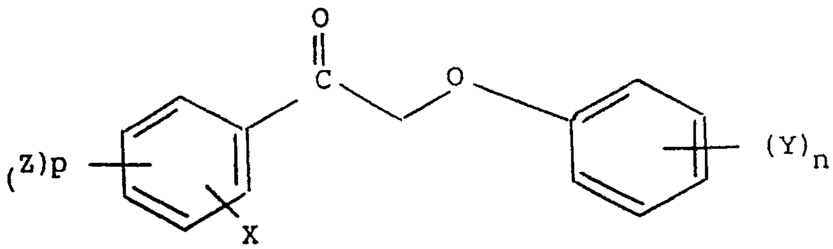

(IV$_A$)

dans laquelle Z, Y, n et p ont les définitions antérieures
et X est un hydroxyle
que l'on réduit ensuite en composé de formule générale I$_A$ par action d'un hydrure mixte de métal alcalin en présence d'un catalyseur métallique ou par réaction avec un chloroformiate d'alcoyle inférieur puis réduction du produit intermédiaire par un borohydrure de métal alcalin
que l'on peut alcoyler par action d'un réactif d'alcoylation en milieu basique ou acyler par action d'un dérivé fonctionnel d'acide carboxylique, sulfonique, sulfurique ou phosphorique.
14°- Un procédé de préparation des composés de formule générale I selon l'une des revendication 1 à 11° caractérisé en ce qu'on bloque la fonction phénol d'une hydroxy-acétophénone de formule générale V

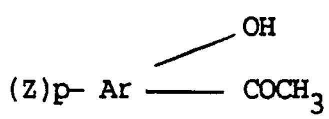

(V)

dans laquelle Ar, Z et p sont définis comme précedemment
et X est un hydroxyle libre
par un réactif aisément clivable, puis soumet le phénol bloqué de formule VI

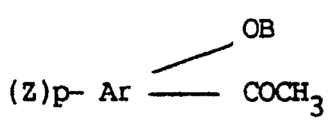

(VI)

dans laquelle B est un radical aisément clivable
Z et p sont définis comme précedemment
que l'on soumet à l'action d'un agent de bromation pour former une $\alpha$-bromocétone de formule VII

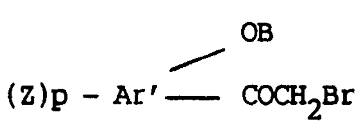

(VII)

dans laquelle Z, Ar et p ont les mêmes significations que précedemment
condense celle-ci avec un phénol de formule

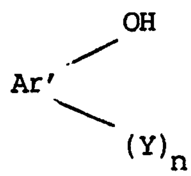

dans laquelle Ar', Y et n sont définis comme précédemment
pour obtenir une phénoxy éthanone de formule générale VIII

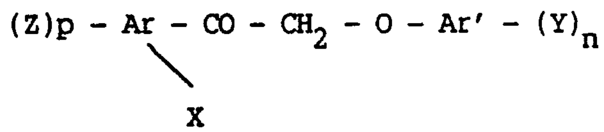 (VIII)

dans laquelle la définition des substituants Y, Z, Ar, n et p demeure inchangée
et X est un radial hydroxy blcqué par une fonction aisément clivable
puis soumet le composé VIII à une hydrogénation en présence d'un catalyseur métallique ou à une acidolyse, pour libérer la fonction phénol en formant une phénoxy éthanone de formule générale IX

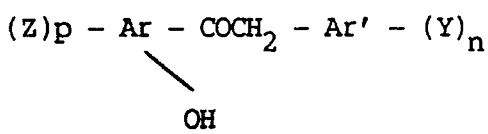 (IX)

dans laquelle Y, Z, n, p, Ar et Ar' sont définis comme précédemment
traite ce dernier par un agent réducteur pour former l'alcool correspondant de formule générale X

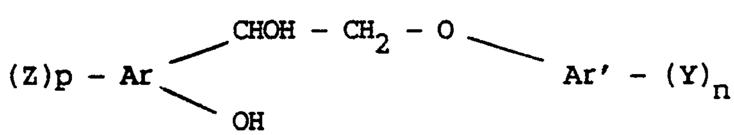 (X)

dans laquelle Z, Y, n et p sont définis comme précedemment
et X est un hydroxyle libre.
15°- Un procédé de préparation des composés de formule générale I dans lequel on bloque la fonction phénol d'une hydroxy acétophénone de formule générale $V_A$

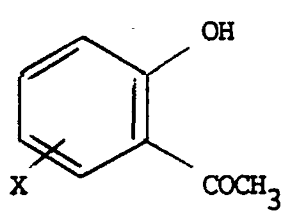 ($V_A$)

par un réactif labile puis soumet à l'action d'un agent de bromation le phénol bloqué, pour former une α-bromocétone, condense la cétone α-bromée ainsi formée avec un phénol pour former une phénoxyéthanone de formule $VI_A$

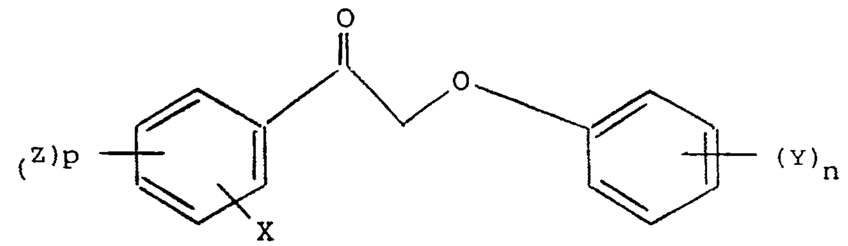

$(VI_A)$

dans laquelle les substituants Y, Z, n et p ont les significations fournies antérieurement et X est une fonction phénol bloquée puis soumet le composé $VI_A$ à une hydrogénation en présence d'un catalyseur métalique ou à une acidolyse pour libérer la fonction phénol en formant une phénoxyéthanone de formule générale $VII_A$

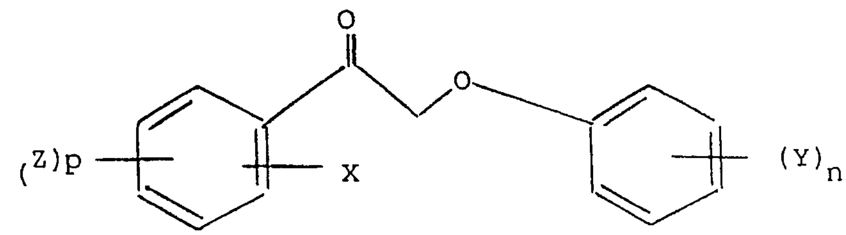

$(VII_A)$

dans laquelle Y, Z, n et p sont définis comme précédemment et X est un hydroxyle puis le composé de formule $VII_A$ est soumis à l'action d'un agent réducteur pour former l'alcool correspondant de formule générale $VIII_A$

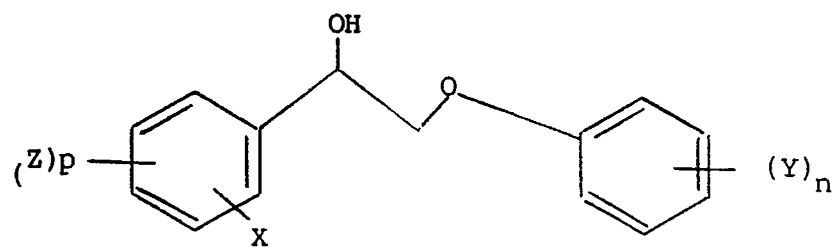

$(VIII_A)$

dans laquelle Z, Y, n et p sont définis comme précédemment et X est un hydroxyle libre

16°- Un procédé d'obtention des composés de formule générale I selon l'une des revendications 1 à 11°, dans laquelle R est un radical alcoyle inférieur, caractérisé en ce que l'on soumet une phénoxy éthanone de formule générale VIII

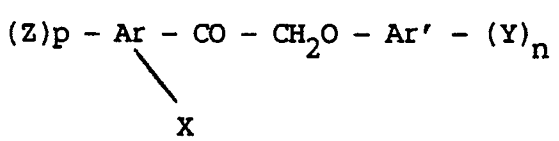

(VIII)

dans laquelle Z, Y, Ar, Ar', p et n sont définis comme précédemment et X est un hydroxyle bloqué à l'action d'un bromure de triarylalcoyl phosphonium dans les conditions de la réaction de WITTIG pour former un dérivé éthylénique de formule générale XI

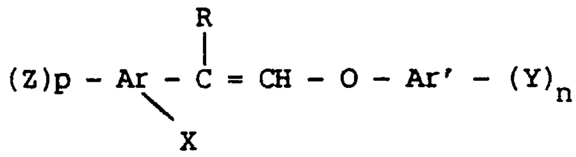

(XI)

dans laquelle Z, Y, Ar, Ar', n et p sont définis comme précédemment
X est une fonction phénol bloquée
et R est un radical alcoyle inférieur
puis soumet ce dernier composé à une hydrogénation en présence d'un catalyseur métallique pour obtenir le composé α-alcoylé de formule générale I

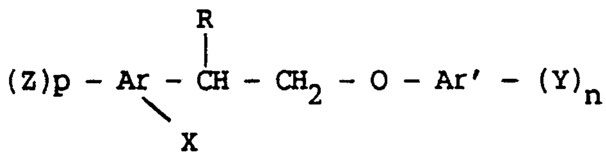

(I)

dans laquelle Z, Y, Ar, Ar', n et p sont définis comme précédemment
X est un hydroxyle libre
et R est un radical alcoyle inférieur
que l'on peut si désiré alcoyler ou acyler dans les conditions définies précédemment.

17°- Un procédé d'obtention des composés de formule générale I dans laquelle R est un radical alcoyle inférieur, selon l'une des revendications 1 à 3° dans lequel un composé de formule générale $VI_A$

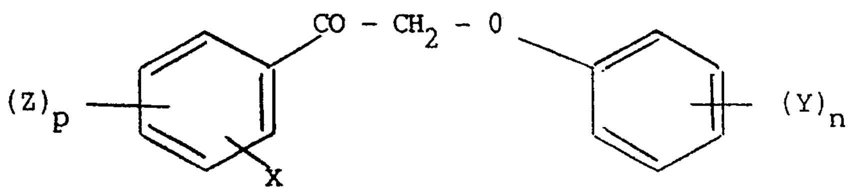

(VIA)

dans laquelle les substituants Y, Z, n et p ont les significations fournies antérieurement et X est une fonction phénol bloquée
est soumis à l'action d'un bromure de triaryl alcoyl phosphonium dans les conditions de la réaction de Wittig pour former un dérivé éthylénique de formule générale $IX_A$

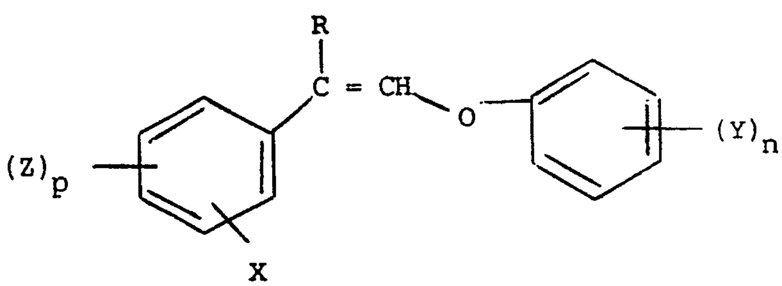

($IX_A$)

dans laquelle Z, Y, n et p ont les significations antérieures
X est une fonction phénol bloquée
et R est un radical alcoyle inférieur
puis soumet ce dernier composé à une hydrogénation en présence d'un catalyseur métallique et obtient un composé α-alcoylé de formule générale $I_A$

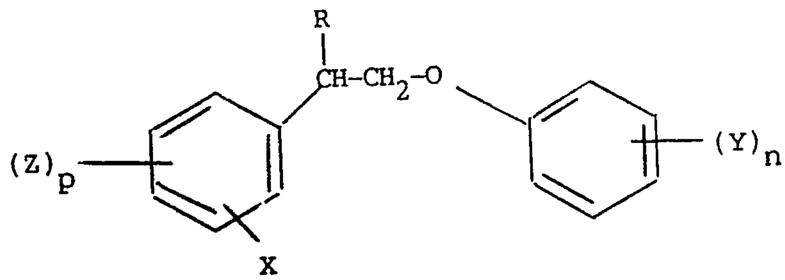

$(I_A)$

dans laquelle Z, Y, n et p sont définis comme précédemment
X est un hydroxy
et R est un radical alcoyle inférieur
que l'on peut, si désiré, alcoyler ou acyler dans les conditions définies précédemment

18° Un procédé d'obtention des composés de formule générale I selon l'une des revendications 1 à 3°, caractérisé en ce que l'on soumet un benzaldéhyde substitué de formule générale XI

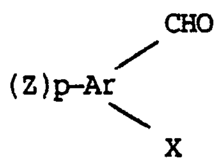

(XI)

dans laquelle Z, p et Ar ont les significations fournies antérieurement
et X est une fonction phénol bloquée
à l'action d'un sel de triaryl phénoxy méthyl phosphonium de formule

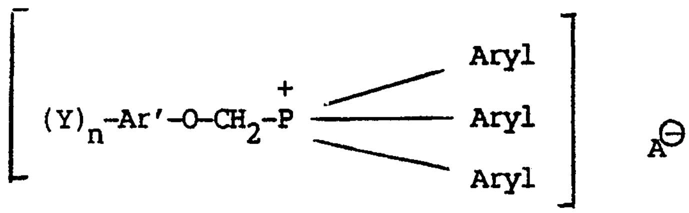

dans laquelle Y, Ar′ et n sont définis comme précédemment
et A est un anion dérivé d'un acide minéral non réactif
dans les conditions de la réaction de WITTIG pour former un dérivé éthylénique de formule gnérale XII

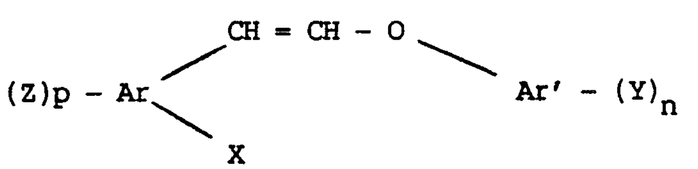

(XII)

dans laquelle Z, Ar, X, Ar′, Y, n et p ont les significations fournies précédemment
que l'on hydrogène par l'hydrogène en présence d'un catalyseur métallique en composé de formule générale I

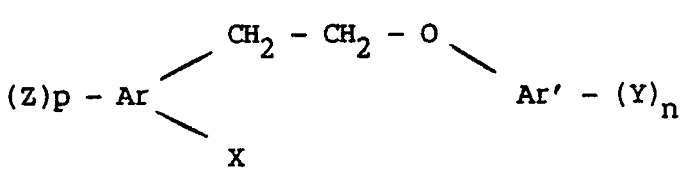

(I)

dans laquelle les substituants Z, Ar, Ar′, Y, n, p et X ont les significations antérieures.

19°- Un procédé d'obtention des composés de formule générale I elon l'une des revendications 1 à 3° dans lequel on soumet un benzaldéhyde substitué de formule générale $X_A$

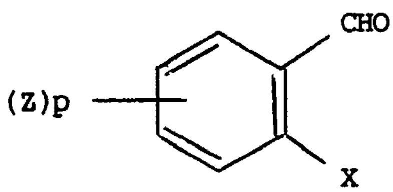

($X_A$)

dans laquelle Z et p ont les définitions antérieures

et X est une fonction phénol bloquée

à l'action d'un sel de triarylphénoxy méthyl phosphonium dans les conditions de la réaction de WITTIG pour former un dérivé éthylénique de formule $XI_A$

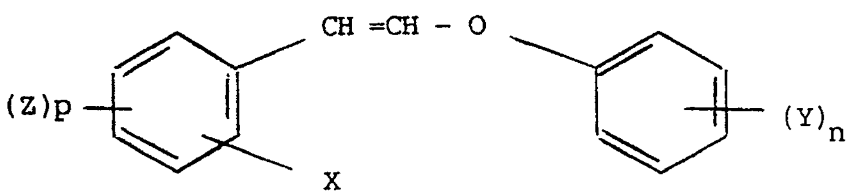

($XI_A$)

dans laquelle Z, Y, n et p ont les significations fournies antérieurement

et X est une fonction phénol bloquée

que l'on hydrogène en composé de formule générale $I_A$ par l'hydrogène en présence d'un catalyseur métallique.

20°- Un procédé d'obtention des composés de formule générale I selon l'une des revendications 1 à 3° caractérisé en ce qu'on bloque les fonctions réactives d'un hydroxyaryl éthanol de formule générale XIII

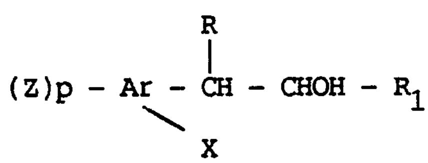

(XIII)

dans laquelle Z, Ar, Ar′, R et I sont définis comme précédemment

et X est un hydroxyle libre

à l'aide d'un réactif aisément clivable, puis fait réagir le dérivé ainsi bloqué avec un phénol de formule générale XIV

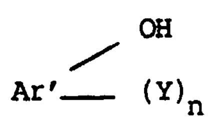

(XIV)

dans laquelle Y, n et Ar′ ont les significations fournies antérieurement

pour fournir un phénoxyéthyl benzène de formule générale XV

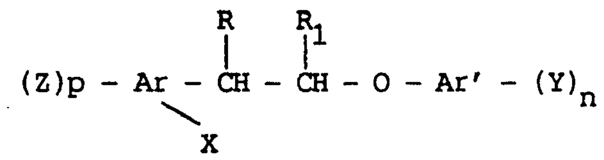

(XV)

dans laquelle Z, Y, Ar, Ar′, n et p sont définis comme précédemment

R et $R_1$, distinctement l'un de l'autre, sont de l'hydrogène ou un radical alcoyle inférieur

et X est un hydroxyle bloqué

dont on libère la fonction hydroxyle par action d'un agent alcalin pour former un composé de formule générale I

21°- Un procédé d'obtention des composés de formule générale I selon l'une des revendications 1 à 3° dans lequel on bloque les fonctions réactives d'un hydroxy phényl éthanol formule générale $XII_A$

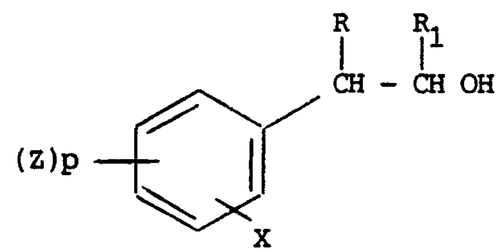

($XII_A$)

dans laquelle Z et p sont définis comme précédemment

X est un hydroxyle

R et $R_1$ sont de l'hydrogène ou un radical alcoyle inférieur

à l'aide d'un réactif aisément clivable, fait réagir le dérivé bloqué avec un phénol de formule générale $XIII_A$

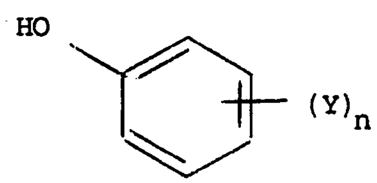

($XIII_A$)

dans laquelle Y et n ont les définitions antérieures

pour obtenir un phényléthoxy phényle de formule générale $XIV_A$

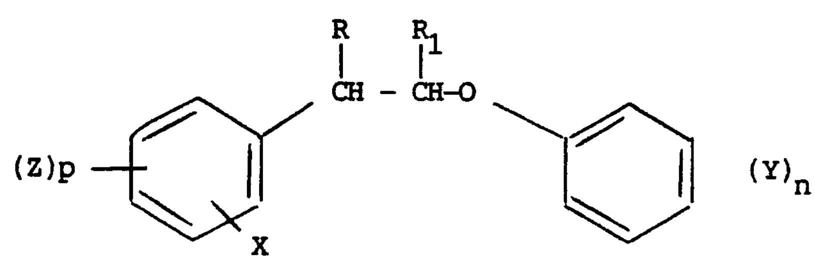

($XIV_A$)

dans laquelle Z, Y, n et p sont définis comme précédemment

R et $R_1$ sont de l'hydrogène ou un radical alcoyle inférieur

et X est un hydroxyle bloqué

puis libère la fonction hydroxyle par action d'un agent alcalin pour former un composé de formule générale $I_A$.

22°- A titre de produits nouveaux, les produits intermédiaires formés au cours de la synthèse, à savoir les dérivés éthyléniques de formule générale $IX_A$

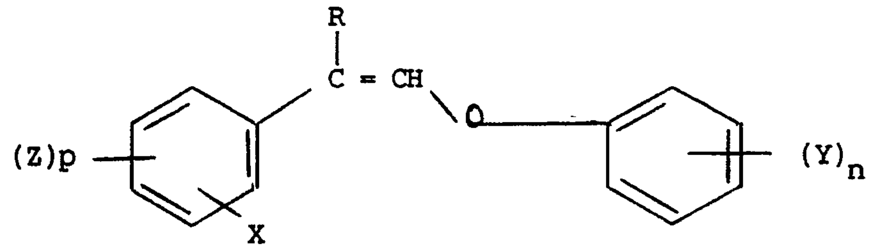

$(IX_A)$

dans laquelle Z, Y, X, R, n et p ont les significations fournies précédemment et les dérivés éthyléniques de formule générale $XII_A$

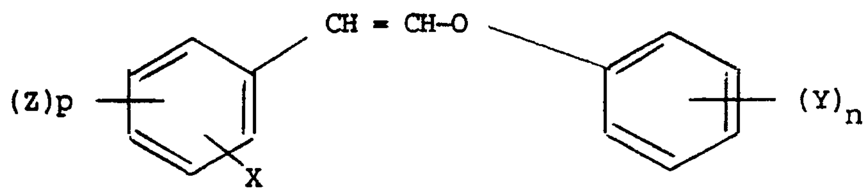

$(XII_A)$

dans laquelle Z, Y, X, n et p sont définis comme précedemment.

23°- Ls compositions pharmaceutiques caractérisées en ce qu'elles renferment à titre de principe actif au moins un composé de formule généraleI selon l'une des revendications 1 à 11°

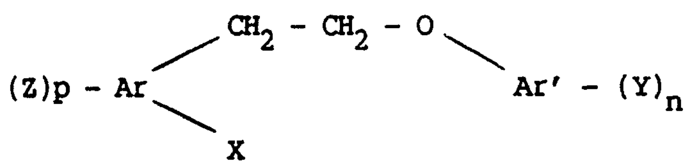

(I)

en association ou en mélange avec un excipient ou avec un véhicule inerte non-toxique pharmaceutiquement-acceptable.

24°- Les compositions pharmaceutiques caractérisées en ce qu'elles renferment à titre de principe actif au moins un composé de formule générale $I_A$

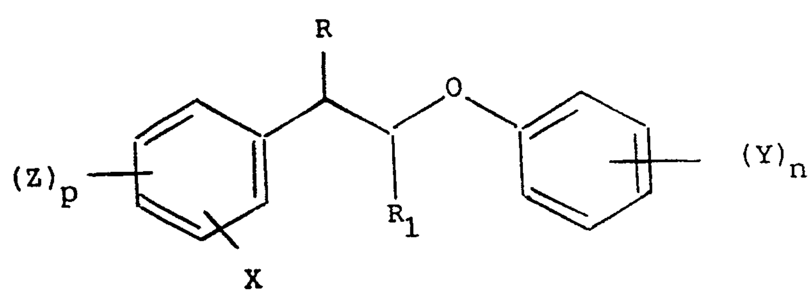

$(I_A)$

dans laquelle X représente un hydroxyle, un acyloxy dont le reste acyle dérive d'un acide carboxylique, sulfonique, phosphorique, de l'acide sulfurique et de l'acide phosphorique, un alcoxy inférieur ou un alcenyloxy inférieur

Y représente de l'hydrogène, un halogène, un alcoylène dioxy, un hydroxy, un alcoxy inférieur, un acyloxy dérivé d'un acide organique carboxylique, un radical alcoyle inférieur, un radical alcoyle inférieur substitué par un alcoxy carbonyle, une chaine hydroxy alcoyle ayant au moins 2 atomes de carbone, un alcoxy carbonyle ou la chaine alcoylidène d'un cycle aromatique ayant 5 ou 6 chainons

Z représente de l'hydrogène, un hydroxyle, un alcoxy, un alcényloxy, un phénoxy, un phénylalcoyle inférieur, un alcoxy inférieur, un acyloxy, un alcoyle inférieur, un alcoyle inférieur substitué par un (alcoyle

inférieur) oxycarbonyle ou par un hydroxy carbonyle, une chaine $CO(CH_2)_m CH_3$ dans laquelle m est un nombre entier alant de 1 à 4, une chaine $CHOH\text{-}(CH_2)_{m'} CH_3$ dans laquele m′ représente un nombre entier variant de 1 à 4, un groupe carboxamido éventuellement substitué par un ou deux radicaux alcoyle inférieur, un phényl ou un phénoxy.

25°- Une composition pharmaceutique selon l'une des revendications 23 ou 24° dans laquelle la teneur en principe actif de formule générale I s'échelonne de 1 à 200 mg par prise unitaire.

26°- Une composition pharmaceutique selon les revendications 23 à 25° dans laquelle le véhicule ou l'excipient sont un de ceux qui conviennent pour l'administration par voie parentérale, digestive, rectale, permuqueuse ou percutanée.

Revendications pour les Etats contractants suivants: GR, ES

1°- Un procédé d'obtention de nouveaux phénoxyéthyl benzènes de formule générale I

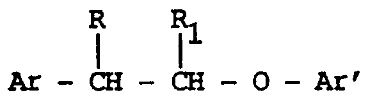

(I)

dans laquelle Ar est un radical aromatique mono- ou bicyclique hydroxylé choisi parmi les dérivés benzéniques de formule

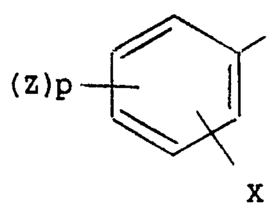

dans laquelle X représente un hydroxyle libre, estérifié par un acide carboxylique, ou un acide sulfonique, par l'acide phosphorique, ou par l'acide sulfurique ou ethérifié par un alcoyle inférieur ou un alcényle inférieur

Z est un substituant choisi dans le groupe constitué par l'hydrogène, les halogènes, un hydroxy, un alcoxy inférieur, un acyloxy, un phényl alcoyle inférieur, un alcoyle inférieur, un alcoyle inférieur substitué par un (alcoyl inférieur)oxycarbonyle ou par un hydroxy carbonyle, une chaine $CO(CH_2)_m CH_3$ dans laquelle m représente un nombre entier allant de 1 à 4, une chaine $CHOH\text{-}(CH_2)_{m'} CH_3$ dans laquelle m′ représente un nombre entier variant de 1 à 4, un phényle, un phénoxy et un groupe carboxamido éventuellement substitué par un ou deux radicaux alcoyle inférieur

et parmi les dérivés naphtaléniques choisis dans le groupe constitué parmi les composés de formule

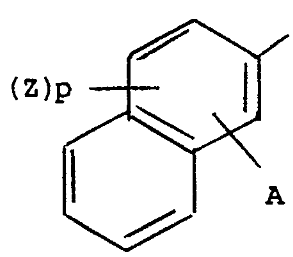

dans laquelle A est un hydroxyle libre, ethérifié ou estérifié

Z est défini comme précédemment

et p est un nombre entier variant de 1 à 3

et ceux de formule

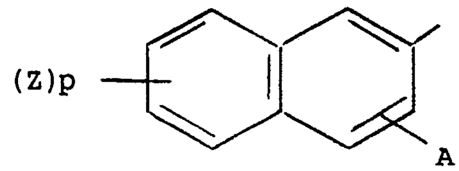

dans laquelle A, Z et p sont définis comme précédemment
Ar′ est un radical aromatique mono- ou bicyclique choisi dans le groupe constitué parmi
- les dérivés benzéniques de formule

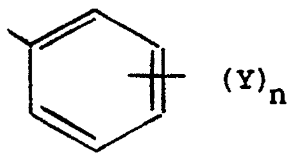

dans laquelle Y représente de l'hydrogène, un halogène, un alcoylène dioxy, un hydroxy, un alcoxy inférieur, un acyloxy dérivé d'un acide organique carboxylique, un alcoyle inférieur, un alcoyle inférieur substitué par un radical (alcoxy inférieur) carbonyle, une chaine oxo alcoyle de formule $CO(CH_2)_m-CH_3$ dans laquelle m représente un nombre entier allant de 1 à 4 et une chaine hydroxy alcoyle de formule $CHOH-(CH_2)_{m'}-CH_3$ dans laquelle m′ représente un nombre entier allant de 1 à 4.
- les dérivés naphtaléniques choisis dans le groupe constitué par les dérivés de formule

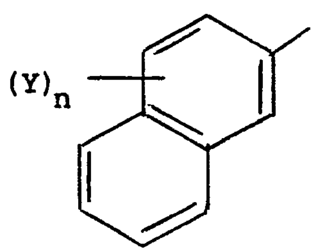

dans laquelle Y est défini comme précédemment
et n est un nombre entier variant de 1 à 3
- et les dérivés de formule

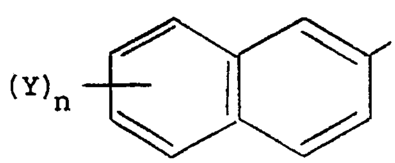

dans laquelle Y est défini comme précedemment
et n est un nombre entier variant de 1 à 3
R est un hydrogène, un hydroxyle, l'oxygène d'une fonction carbonylée ou un radical alcoyle inférieur
$R_1$ est un hydrogène ou un radical alcoyle inférieur caractérisé en ce que l'on condense un phénol de formule générale II
Ar - X (II)
dans laquelle Ar, X et Z sont définis comme précédemment
avec un aryl acétonitrile de formule générale III
Ar′ - O-$CH_2$ CN (III)
dans laquelle Ar′ est défini comme précédemment
en présence d'un catalyseur acide pour former une diaryl éthanone de formule générale IV

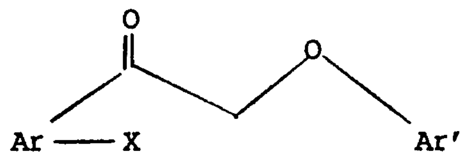

(IV)

dans laquelle Ar et Ar′ sont définis comme précedemment
et X est un hydroxyle libre
que l'on réduit ensuite par action d'un hydrure mixte de métal alcalin en présence d'un catalyseur métallique ou par réaction avec un chloroformiate d'alcoyle inférieur puis réduction du produit formé intermédiairement, par un borohydrure de métal alcalin pour obtenir un composé de formule générale I
2°- Un procédé d'obtention des composés de formule générale I selon la revendication 1° qui consiste en ce que l'on condense un phénol de formule générale $II_A$

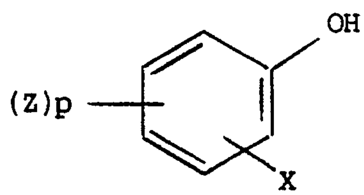

$(II_A)$

dans laquelle X, Z et p sont éfinis comme précedemment
avec un phénoxy acéto-nitrile de formule générale $III_A$

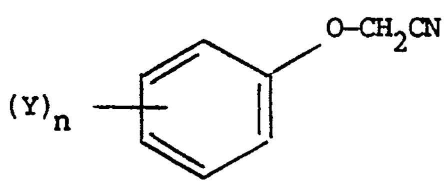

$(III_A)$

dans laquelle Y et n ont les définitions antérieures
en présence d'un catalyseur acide, pour former l'éthanone de formule générale $IV_A$

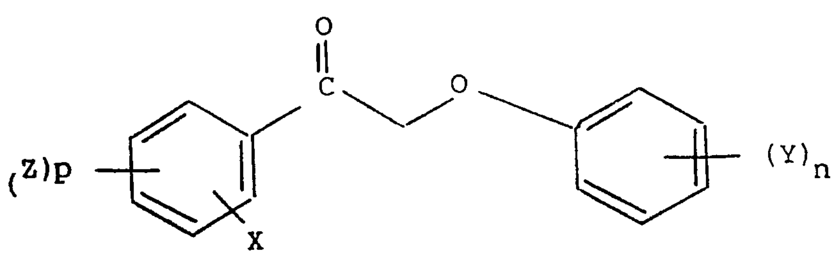

$(IV_A)$

dans laquelle Z, Y, n et p ont les définitions antérieures
et X est un hydroxyle
que l'on réduit ensuite en composé de formule générale $I_A$ par action d'un hydrure mixte de métal alcalin en présence d'un catalyseur métallique ou par réaction avec un chloroformiate d'alcoyle inférieur puis réduction du produit intermédiaire par un borohydrure de métal alcalin
que l'on peut alcoyler par action d'un réactif d'alcoylation en milieu basique ou acyler par action d'un dérivé fonctionnel d'acide carboxylique, sulfonique, sulfurique ou phosphorique.
3° Un procédé d'obtention des formes optiquement-actives des composés formule générale I lorsque R ou $R_1$ sont différents de l'hydrogène et que R ou $R_1$ représentent un hydroxyle ou un radical alcoyle inférieur

qui consiste en ce que l'on fait réagir un composé selon la revendication 1° ou la revendication 2° avec un réactif chiral et sépare le dérivé désiré.

4°- Un procédé d'obtention des sels avec un métal alcalin ou alcalino terreux ou avec une base organique des composés de formule I lorsque X représente un hydroxyle estérifié par l'acide phosphorique ou sulfurique, qui consiste à mettre en contact ledit composé de formule I avec une base minérale ou organique.

5°- Un procédé de préparation des composés de formule générale I selon l'une des revendications 1 à 4° caractérisé en ce qu'on bloque la fonction phénol d'une hydroxy-acétophénone de formule générale V

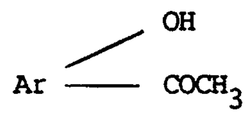

(V)

dans laquelle Ar est défini comme précedemment
et X est un hydroxyle libre
par un réactif aisément clivable, puis soumet le phénol bloqué de formule VI

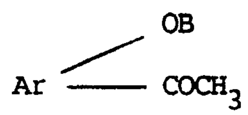

(VI)

dans laquelle B est un radical aisément clivable
Ar est défini comme précedemment
que l'on soumet à l'action d'un agent de bromation pour former une α-bromocétone de formule VII

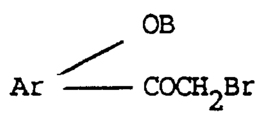

(VII)

dans laquelle Ar a les mêmes significations que précedemment
condense celle-ci avec un phénol de formule

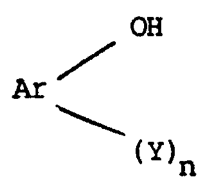

dans laquelle Y et n sont définis comme précédemment
pour obtenir une phénoxy éthanone de formule générale VIII

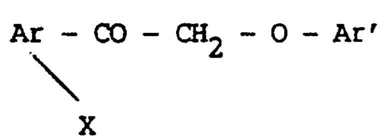

(VIII)

dans laquelle la définition de Ar et de Ar′ demeure inchangée et X est un radial hydroxy bloqué par une fonction aisément clivable
puis soumet le composé VIII à une hydrogénation en présence d'un catalyseur métallique ou à une acidolyse, pour libérer la fonction phénol en formant une phénoxy éthanone de formule générale IX

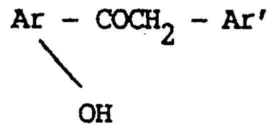

(IX)

dans laquelle Ar et Ar' sont définis comme précédemment
traite cette dernière par un agent réducteur pour former l'alcool correspondant de formule générale X

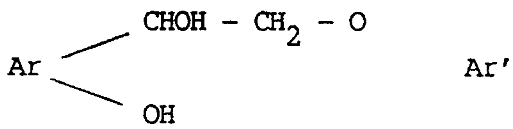

(X)

dans laquelle Ar et Ar' sont définis comme précedemment
et X est un hydroxyle libre.
6°- Un procédé de préparation des composés de formule générale I selon l'une des revendications 1 à 4° dans lequel on bloque la fonction phénol d'une hydroxy acétophénone de formule générale $V_A$

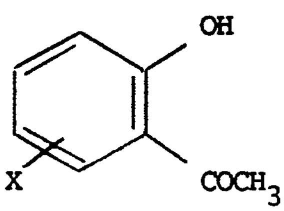

($V_A$)

par un réactif labile puis soumet à l'action d'un agent de bromation le phénol bloqué, pour former une α-bromocétone, condense la cétone α-bromée ainsi formée avec un phénol pour former une phénoxyéthanone de formule $VI_A$

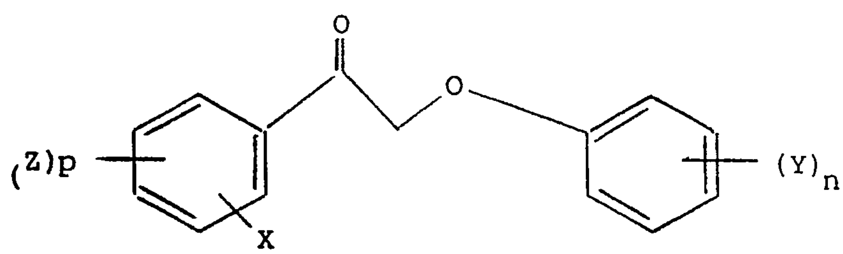

($VI_A$)

dans laquelle les substituants Y, Z, n et p ont les significations fournies antérieurement
et X est une fonction phénol bloquée
puis soumet le composé $VI_A$ à une hydrogénation en présence d'un catalyseur métalique ou à une acidolyse pour libérer la fonction phénol en formant une phénoxyéthanone de formule générale $VII_A$

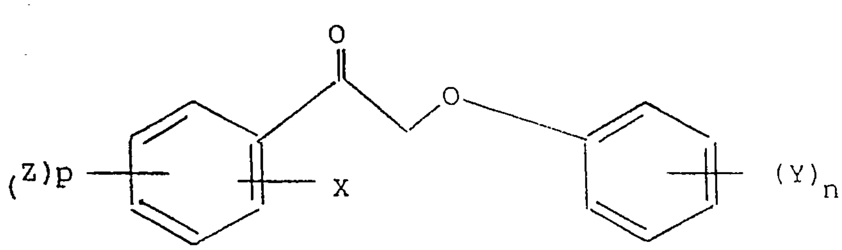

($VII_A$)

dans laquelle Y, Z, n et p sont définis comme précédemment
et X est un hydroxyle
puis le composé de formule $VII_A$ est soumis à l'action d'un agent réducteur pour former l'alcool correspondant de formule générale $VIII_A$

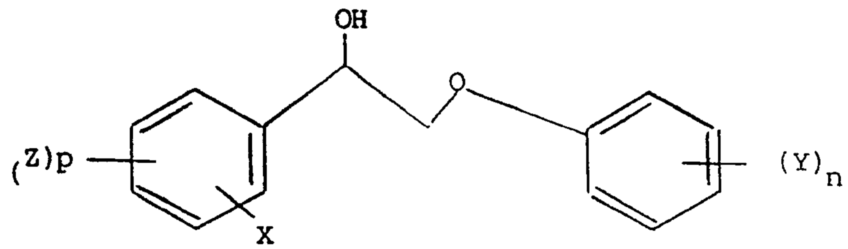

$(VIII_A)$

dans laquelle Z, Y, n et p sont définis comme précédemment et X est un hydroxyle libre
7°- Un procédé d'obtention des composés de formule générale I selon l'une des revendications 1 à 4°, dans laquelle R est un radical alcoyle inférieur, caractérisé en ce que l'on soumet une phénoxy éthanone de formule générale VIII

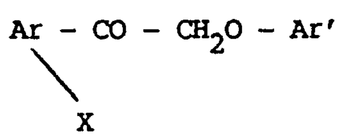

(VIII)

dans laquelle Ar et Ar′ sont définis comme précédemment
et X est un hydroxyle bloqué
à l'action d'un bromure de triarylalcoyl phosphonium dans les conditions de la réaction de WITTIG pour former un dérivé éthylénique de formule générale XI

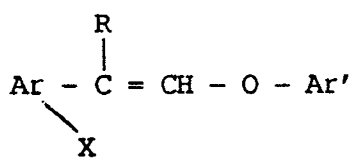

(XI)

dans laquelle Ar et Ar′ sont définis comme précédemment
X est une fonction phénol bloquée
et R est un radical alcoyle inférieur
puis soumet ce dernier composé à une hydrogénation en présence. d'un catalyseur métallique pour obtenir le composé α-alcoylé de formule générale I

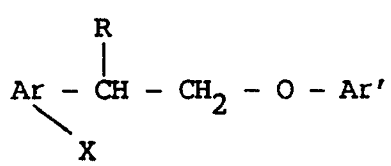

(I)

dans laquelle Ar et Ar′ sont définis comme précédemment
X est un hydroxyle libre
et R est un radical alcoyle inférieur
que l'on peut si désiré alcoyler ou acyler dans les conditions définies précédemment.
8°- Un procédé d'obtention des composés de formule générale I dans laquelle R est un radical alcoyle inférieur, selon l'une des revendications 1 à 4° dans lequel un composé de formule générale $VI_A$

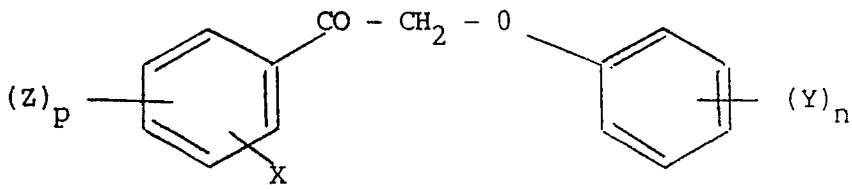

$(VI_A)$

dans laquelle les substituants Y, Z, n et p ont les significations fournies antérieurement et X est une fonction phénol bloquée est soumis à l'action d'un bromure de triaryl alcoyl phosphonium dans les conditions de la réaction de Wittig pour former un dérivé éthylénique de formule générale $IX_A$

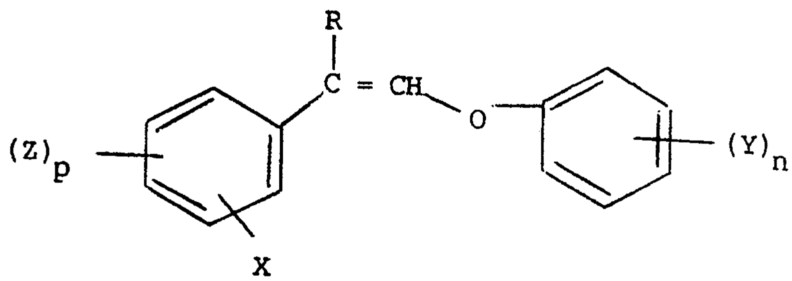

$(IX_A)$

dans laquelle Z, Y, n et p ont les significations antérieures
X est une fonction phénol bloquée
et R est un radical alcoyle inférieur
puis soumet ce dernier composé à une hydrogénation en présence d'un catalyseur métallique et obtient un composé α-alcoylé de formule générale $I_A$

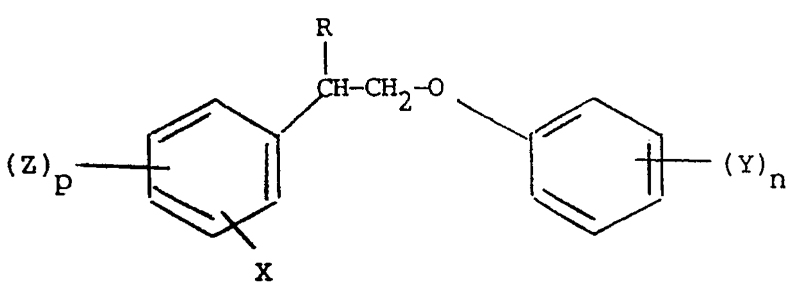

$(I_A)$

dans laquelle Z, Y, n et p sont définis comme précédemment
X est un hydroxy
et R est un radical alcoyle inférieur
que l'on peut, si désiré, alcoyler ou acyler dans les conditions définies précédemment

9°- Un procédé d'obtention des composés de formule générale I selon l'une des revendications 1 à 4°, caractérisé en ce que l'on soumet un benzaldéhyde substitué de formule générale XI

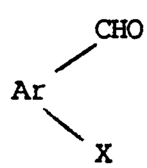

(XI)

dans laquelle Ar a les significations fournies antérieurement
et X est une fonction phénol bloquée
à l'action d'un sel de triaryl phénoxy méthyl phosphonium de formule

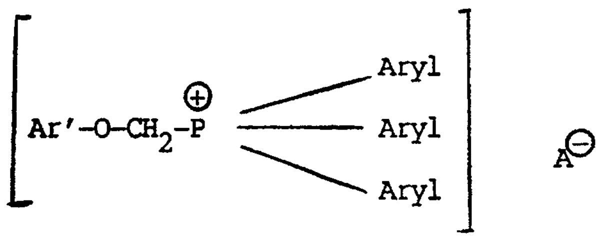

dans laquelle Ar′ est défini comme précédemment
et A est un anion dérivé d'un acide minéral non réactif dans les conditions de la réaction de WITTIG pour former un dérivé éthylénique de formule gnérale XII

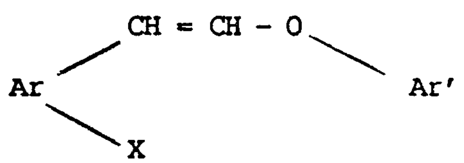

(XII)

dans laquelle Ar, X et Ar′ ont les significations fournies précédemment
que l'on hydrogène par l'hydrogène en présence d'un catalyseur métallique en composé de formule générale I

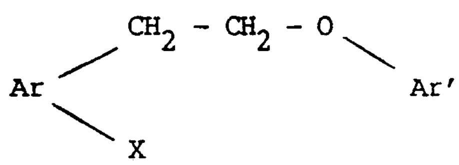

(I)

dans laquelle les substituants Z, Ar, Ar′, Y, n, p et X ont les significations antérieures.

10°- Un procédé d'obtention des composés de formule générale I elon l'une des revendications 1 à 4° dans lequel on soumet un benzaldéhyde substitué de formule générale $X_A$

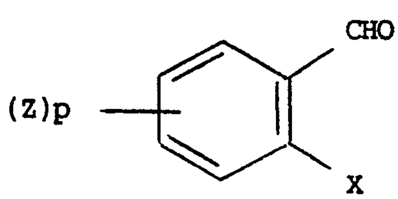

($X_A$)

dans laquelle Z et p ont les définitions antérieures
et X est une fonction phénol bloquée
à l'action d'un sel de triarylphénoxy méthyl phosphonium dans les conditions de la réaction de WITTIG pour former un dérivé éthylénique de formule $XI_A$

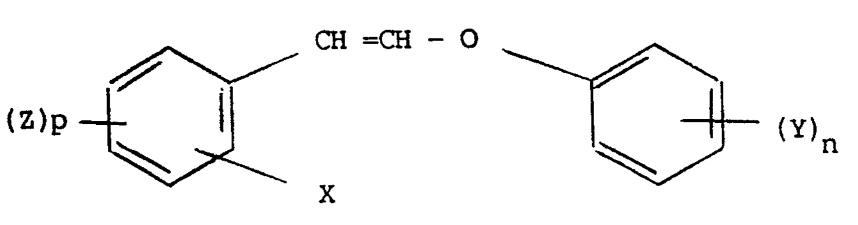

($XI_A$)

dans laquelle Z, Y, n et p ont les significations fournies antérieurement
et X est une fonction phénol bloquée
que l'on hydrogène en composé de formule générale $I_A$ par l'hydrogène en présence d'un catalyseur métallique.

11° Un procédé d'obtention des composés de formule générale I selon l'une des revendications 1 à 4° caractérisé en ce qu'on bloque les fonctions réactives d'un hydroxyaryl éthanol de formule générale XIII

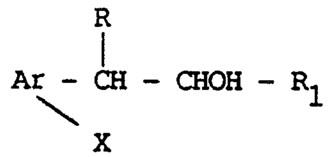

(XIII)

dans laquelle Ar, R et $R_1$ sont définis comme précédemment
et X est un hydroxyle libre
à l'aide d'un réactif aisément clivable, puis fait réagir le dérivé ainsi bloqué avec un phénol de formule générale XIV

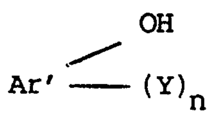

(XIV)

dans laquelle Ar′ a les significations fournies antérieurement
pour fournir un phénoxyéthyl benzène de formule générale XV

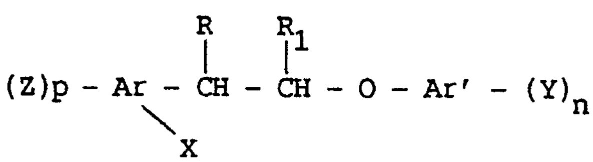

(XV)

dans laquelle Z, Y, Ar, Ar′, n et p sont définis comme précédemment
R et $R_1$, distinctement l'un de l'autre, sont de l'hydrogène ou un radical alcoyle inférieur
et X est un hydroxyle bloqué
dont on libère la fonction hydroxyle par action d'un agent alcalin pour former un composé de formule générale I

12°- Un procédé d'obtention des composés de formule générale I selon l'une des revendications 1 à 4° dans lequel on bloque les fonctions réactives d'un hydroxy phényl éthanol de formule générale $XII_A$

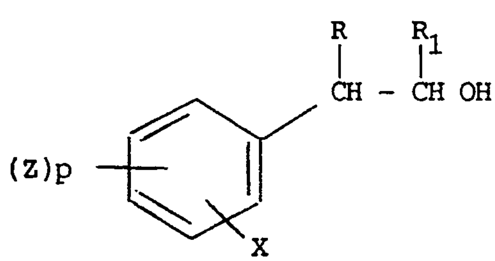

($XII_A$)

dans laquelle Z et p sont définis comme précédemment
X est un hydroxyle
R et $R_1$ sont de l'hydrogène ou un radical alcoyle inférieur
à l'aide d'un réactif aisément clivable, fait réagir le dérivé bloqué avec un phénol de formule générale $XIII_A$

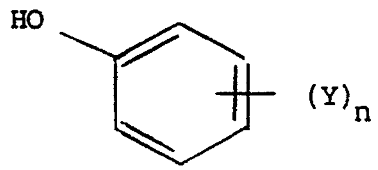

$(XIII_A)$

dans laquelle Y et n ont les définitions antérieures
pour obtenir un phényléthoxy phényle de formule générale $XIV_A$

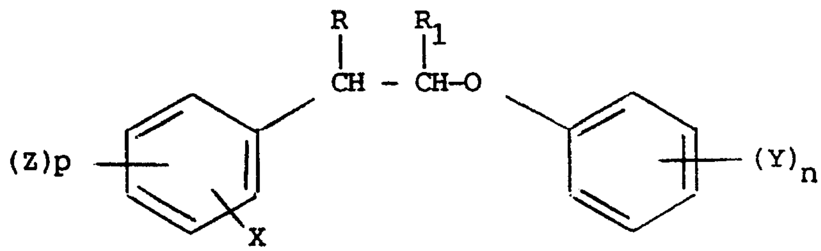

$(XIV_A)$

dans laquelle Z, Y, n et p sont définis comme précédemment
R et $R_1$ sont de l'hydrogène ou un radical alcoyle inférieur
et X est un hydroxyle bloqué
puis libère la fonction hydroxyle par action d'un agent alcalin pour former un composé de formule générale $I_A$.

13°- A titre de produits nouveaux, les produits intermédiaires formés au cours de la synthèse, à savoir les dérivés éthyléniques de formule générale $IX_A$

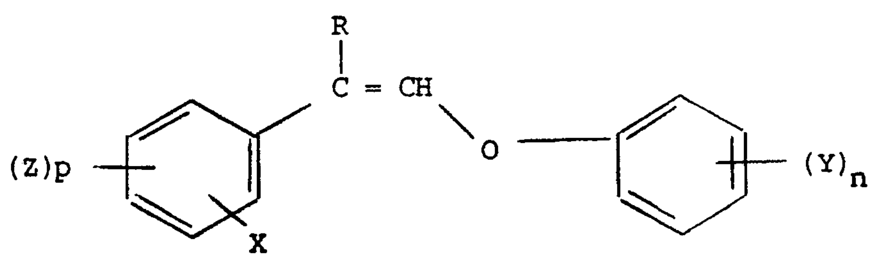

$(IX_A)$

dans laquelle Z, Y, X, R, n et p ont les significations fournies précédemment
et les dérivés éthyléniques de formule générale $XII_A$

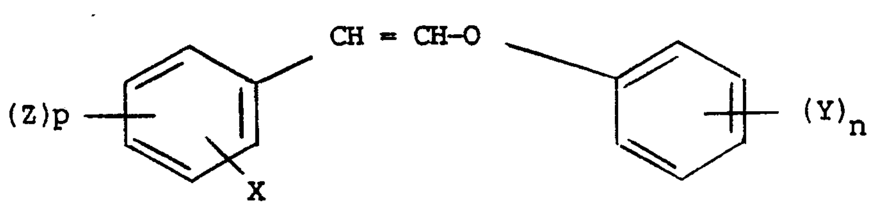

$(XII_A)$

dans laquelle Z, Y, X, n et p sont définis comme précedemment.

14°- Un procédé d'obtention des compositions pharmaceutiques caractérisé en ce qu'on mélange au moins un composé de formule généralel selon l'une des revendications 1 à 4°

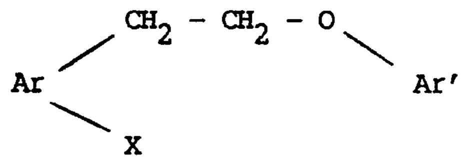

(I)

avec un excipient ou avec un véhicule inerte non-toxique pharmaceutiquement-acceptable.

15°- Un procédé d'obtention des compositions pharmaceutiques selon la revendication 14 caractérisé en ce qu'il renferme à titre de principe actif au moins un composé de formule générale $I_A$

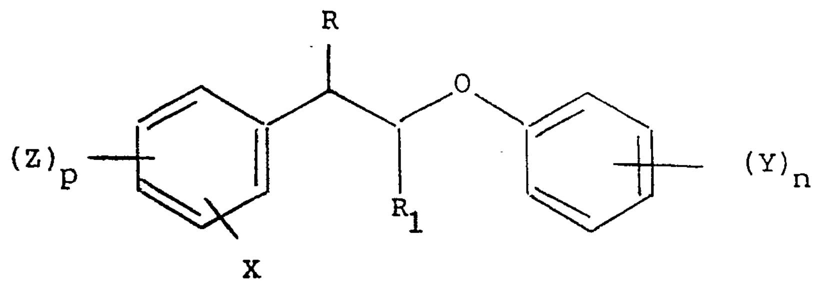

$(I_A)$

dans laquelle X représente un hydroxyle, un acyloxy dont le reste acyle dérive d'un acide carboxylique, sulfonique, phosphorique, de l'acide sulfurique et de l'acide phosphorique, un alcoxy inférieur ou un alcenyloxy inférieur

Y représente de l'hydrogène, un halogène, un alcoylène dioxy, un hydroxy, un alcoxy inférieur, un acyloxy dérivé d'un acide organique carboxylique, un radical alcoyle inférieur, un radical alcoyle inférieur substitué par un alcoxy carbonyle, une chaine hydroxy alcoyle ayant au moins 2 atomes de carbone, un alcoxy carbonyle ou la chaine alcoylidène d'un cycle aromatique ayant 5 ou 6 chainons

Z représente de l'hydrogène, un hydroxyle, un alcoxy, un alcényloxy, un phénoxy, un phénylalcoyle inférieur, un alcoxy inférieur, un acyloxy, un alcoyle inférieur, un alcoyle inférieur substitué par un (alcoyle inférieur) oxycarbonyle ou par un hydroxy carbonyle, une chaine $CO(CH_2)_{m'} CH_3$ dans laquelle m est un nombre entier alant de 1 à 4, une chaine $CHOH-(CH_2)_{m'} CH_3$ dans laquele m' représente un nombre entier variant de 1 à 4, un groupe carboxamido éventuellement substitué par un ou deux radicaux alcoyle inférieur, un phényl ou un phénoxy.

16°- Un procédé d'obention des compositions pharmaceutiques selon l'une des revendications 14 ou 15° dans laquelle la teneur en principe actif de formule générale I s'échelonne de 1 à 200 mg par prise unitaire.

17°- Un procédé d'obtention d'une composition pharmaceutique selon les revendications 14 à 16° dans laquelle le véhicule ou l'excipient sont un de ceux qui conviennent pour l'administration par voie parentérale, digestive, rectale, permuqueuse ou percutanée.

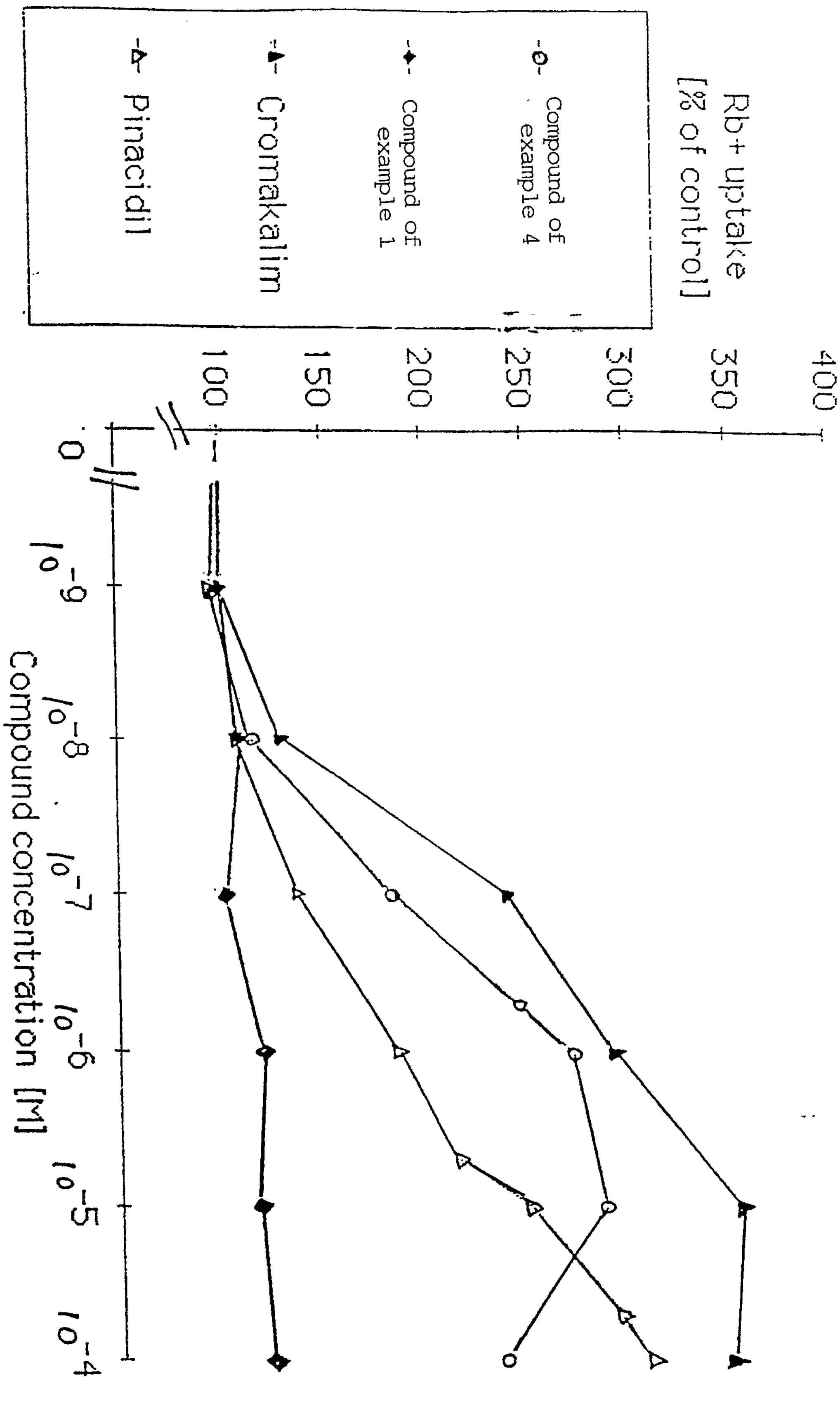
Rb+ uptake
[% of control]
Compound of example 4
Compound of example 1
Cromakalim
Pinacidil
400
350
300
250
200
150
100
0
$10^{-9}$
$10^{-8}$
$10^{-7}$
$10^{-6}$
$10^{-5}$
$10^{-4}$
Compound concentration [M]

Figure 1

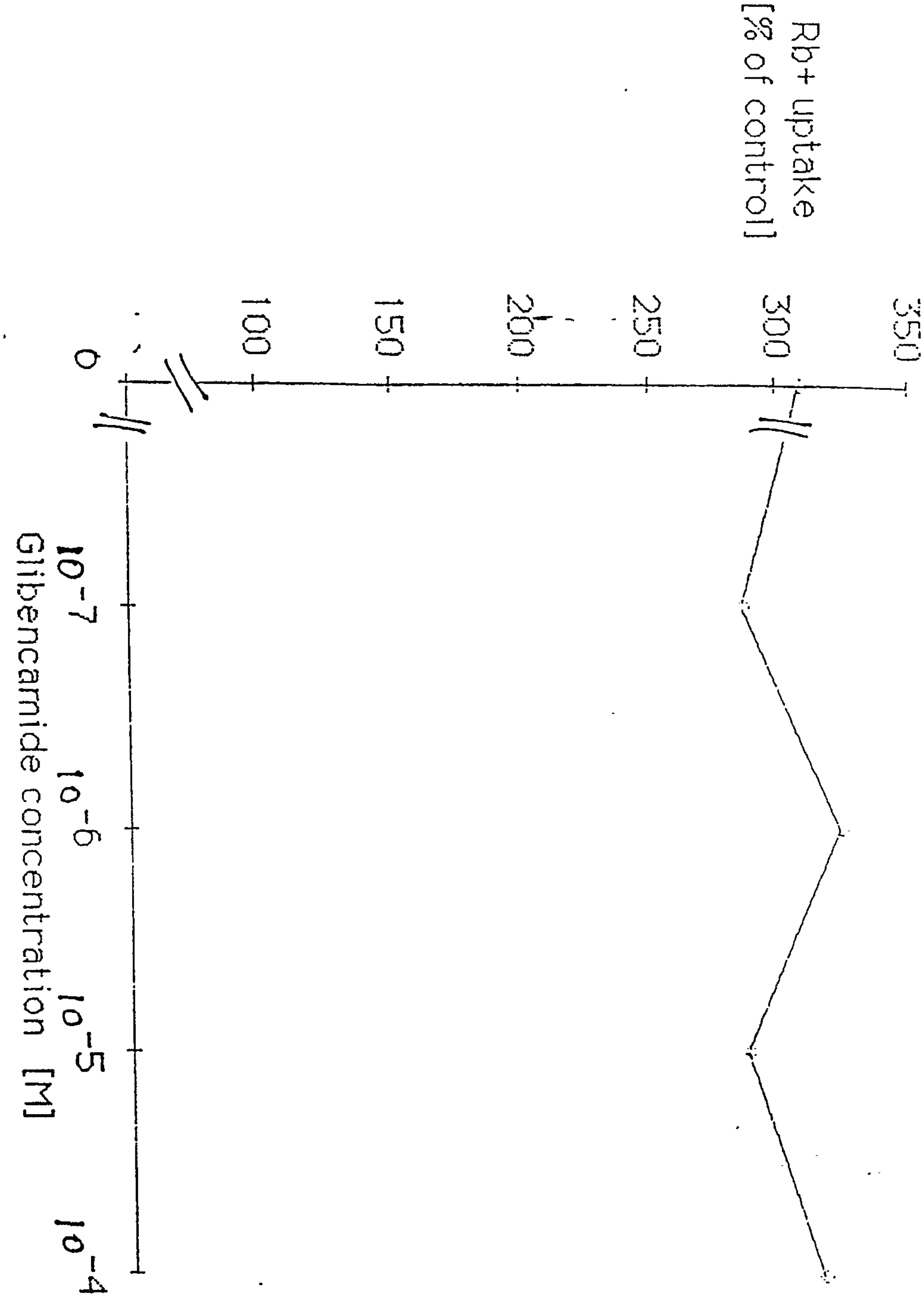


COMPOUND OF EXAMPLE 4 = $10^{-5}$M

Figure 2 -